# EUROPEAN PATENT SPECIFICATION

(11) **EP 1 025 231 B1**
(45) Date of publication and mention of the grant of the patent: **16.07.2003**
(21) Application number: 98953776.6
(22) Date of filing: 20.10.1998
(51) Int. Cl.: C12N 15/18, C07K 14/475, C07K 16/22

(54) **ROBO: A FAMILY OF POLYPEPTIDES AND NUCLEIC ACIDS INVOLVED IN NERVE CELL GUIDANCE**
ROBO: EINE FAMILIE POLYPEPTIDEN UND NUKLEINESÄUREN WIRKSAM IN NERVENZELLLEITUNG
ROBO : UNE FAMILLE DE POLYPEPTIDES ET D'ACIDES NUCLEIQUES IMPLIQUES DANS LE GUIDAGE DES NERFS

(30) Priority: 20.10.1997 US 62921 P; 14.11.1997 US 971172
(43) Date of publication of application: 09.08.2000
(73) Proprietor: The Regents of the University of California, Oakland, CA 94607-5200 (US)
(72) Inventor: GOODMAN, Corey, S., University of California,, Berkeley, CA 94720 (US); KIDD, Thomas, University of California,Berkeley, Berkeley, CA 94720 (US); MITCHELL, Kevin, J. Univ.of California,Berkeley, Berkeley, CA 94720 (US); TEAR, Guy, Imperial College, London SW7 2AZ (GB)
(74) Representative: Goldin, Douglas Michael
(86) International application number: US9822164
(87) International publication number: WO99020764

(56) References cited:
- WO-A-95/13367
- EMEST DATABASE Accession number AA499193 03-JUL-1997 (Rel. 52, Created) Marra M. ET AL. XP002094384
- TEAR G ET AL: "To cross or not to cross: a genetic analysis of guidance at the midline." PERSPECT DEV NEUROBIOL, 1993, 1 (4) P183-94, XP002094379 UNITED STATES
- EMEST DATABASE Accession number AA263962 21-MAR-1997 (Rel. 51, Created) Harvey D. ET AL. XP002094385
- EMEST DATABASE Accession number C68275 20-SEP-1997 (Rel. 52, Created) Kohara Y. ET AL. XP002094386
- EMEST DATABASE Accession number H52936 22-SEP-1995 (Rel. 45, Created) Hillier L. ET AL XP002094387
- EMEST DATABASE Accession number H19148 02-JUL-1995 (Rel. 44, Created) Hillier L. ET AL. XP002094388
- KOLODZIEJ PA ET AL: "FRAZZLED ENCODES A DROSOPHILA MEMBER OF THE DCC IMMUNOGLOBULIN SUBFAMILY AND IS REQUIRED FOR CNS AND MOTOR AXON GUIDANCE" CELL, 1996, 87, 197-204, XP002094380
- SEEGER M ET AL: "Mutations affecting growth cone guidance in Drosophila: genes necessary for guidance toward or away from the midline." NEURON, MAR 1993, 10 (3) P409-26, XP002094381 UNITED STATES
- TEAR G ET AL: "commissureless controls growth cone guidance across the CNS midline in Drosophila and encodes a novel membrane protein." NEURON, MAR 1996, 16 (3) P501-14, XP002094382 UNITED STATES
- KIDD T ET AL: "Roundabout controls axon crossing of the CNS midline and defines a novel subfamily of evolutionarily conserved guidance receptors." CELL, JAN 23 1998, 92 (2) P205-15, XP002094383 UNITED STATES

## Description

### Field of the Invention

The field of this invention is proteins involved in nerve cell guidance.

### Background

Bilaterally symmetric nervous systems, such as those found in insects and vertebrates, have special midline structures that establish a partition between the two mirror image halves. Axons that link the two sides of the nervous system project toward and across the midline, forming axon commissures. These commissural axons project toward the midline, at least in part, by responding to long-range chemoattractants emanating from the midline. One important class of midline chemoattractants are the netrins (Serafini et al., 1994; Kennedy et at., 1994), guidance signals whose structure; function, and midline expression is evolutionarily conserved from nematodes and fruit flies to vertebrates (Hedgecock et al., 1990; Wadsworth et al., 1996; Mitchell et al., 1996; Harris et al., 1996). The attractive actions of netrins appear to be mediated by growth cone receptors of the DCC subfamily of the immunoglobulin (Ig) superfamily (Keino-Masu et al., 1996; Chan et al., 1996; Kolodziej et al., 1996).

The midline also provides important short-range guidance signals. This is best illustrated by considering the different classes of axon projections in the spinal cord of vertebrates or the nerve cord of insects. Although some growth cones extend away from the midline, most extend towards or along the midline during some segment of their trajectory. Certain classes of growth cones either extend towards the midline or longitudinally along it and yet never cross it. Most growth cones (∼90% in the Drosophila CNS), however, do cross the midline. After crossing, the majority of these growth cones turn to project longitudinally, growing along or near the midline. Interestingly, these axons never cross the midline again, despite navigating in the vicinity of other axons that continue to cross.

What midline signals and growth cone receptors control whether growth cones do or do not cross the midline? After crossing once, what mechanism prevents these growth cones from crossing again? Studies in the chick (Stoeckli and Landmesser, 1995; Stoeckli et al., 1997) and grasshopper (Myers and Bastiani, 1993) embryos have led to the suggestion that the midline contains a contact-mediated repellent, and that commissural growth cones must overcome this repellent to cross the midline. For example, this notion that the midline can be repulsive even to growth cones that cross it is supported by time-lapse imaging of the first commissural growth cone in the grasshopper embryo. On contacting the midline, this growth cone often abruptly retracts, although ultimately it overcomes the repulsion and crosses the midline.

One approach to find the genes encoding the components of such a midline guidance system is to screen for mutations in which either too many or too few axons cross the midline. Such a large-scale mutant screen was previously conducted in Drosophila and led to the identification of two key mutations: *commissureless* (*comm*) and *roundabout (robo)* (Seeger et al., 1993; reviewed by Tear et al., 1993). In *comm* mutant embryos, commissural growth cones initially orient toward the midline but then fail to cross it and instead recoil and extend on their own side. *comm* encodes a novel surface protein expressed on midline cells. As commissural growth cones contact and traverse the CNS midline, Comm protein is apparently transferred from midline cells to commissural axons (Tear et al., 1996). In *robo* mutant embryos, many growth cones that normally extend only on their own side instead now project across the midline, and axons that normally cross the midline only once instead appear to cross and recross multiple times (Seeger et al, 1993; Kidd et al., 1997). Double mutants of *comm* and *robo* display a *robo*-like phenotype.

Here we disclose the characterization of *robo* across animal species. *robo* encodes a new class of guidance receptor with 5 Ig domains, 3 fibronectin (FN) type III domains, a transmembrane domain, and a long cytoplasmic domain. Robo defines a new subfamily of Ig superfamily proteins that is highly conserved from fruit flies to mammals. The results of protein expression and transgenic rescue experiments indicate that Robo functions as the gatekeeper controlling midline crossing and that Robo responds to an unknown midline repellent.

### SUMMARY OF THE DISCLOSURE

The disclosure herein provides methods and compositions relating to Robo1 and Robo2, (collectively Robo) polypeptides, related nucleic acids, polypeptide domains thereof having Robo-specific structure and activity, and modulators of Robo function. Robo polypeptides can regulate cell, especially nerve cell, function and morphology. The polypeptides may be produced recombinantly from transformed host cells from the subject Robo polypeptide encoding nucleic acids or purified from mammalian cells. The disclosure provides isolated Robo hybridization probes and primers capable of specifically hybridizing with natural Robo genes, Robo-specific binding agents such as specific antibodies, and methods of making and using the subject compositions in diagnosis (e.g. genetic hybridization screens for Robo transcripts), therapy (e.g. Robo inhibitors to promote nerve cell growth) and in the biopharmaceutical industry (e.g. as immunogens, reagents for isolating Robo genes and polypeptides, reagents for screening chemical libraries for lead pharmacological agents, etc.).

### BRIEF DESCRIPTION OF THE FIGURES

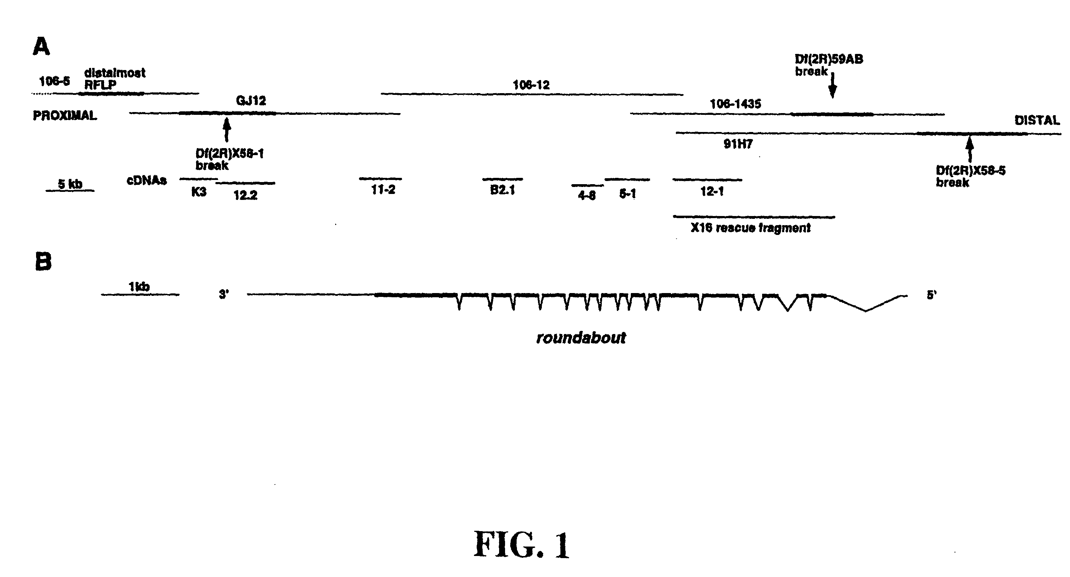

Figure 1 Organization of the roundabout Genomic Locus
   (A) Cosmid chromosome walk through the 58F/59A region of the 2nd chromosome. The position of deficiency breakpoints within the cosmids used are shown in the top two rows. Identified transcripts from the walk are shown below the cosmids. The 12-1 transcript corresponds to the *robo* gene; the direction of transcription is distal to proximal. The location of the 16kb XbaI genomic rescue fragment is indicated below.
   (B) Position and size of introns within the *robo* transcript. Coding sequence is indicated by the thicker part of the line. Introns are represented by gaps. The transcript is shown 3'-5' to reflect its orientation in (A).

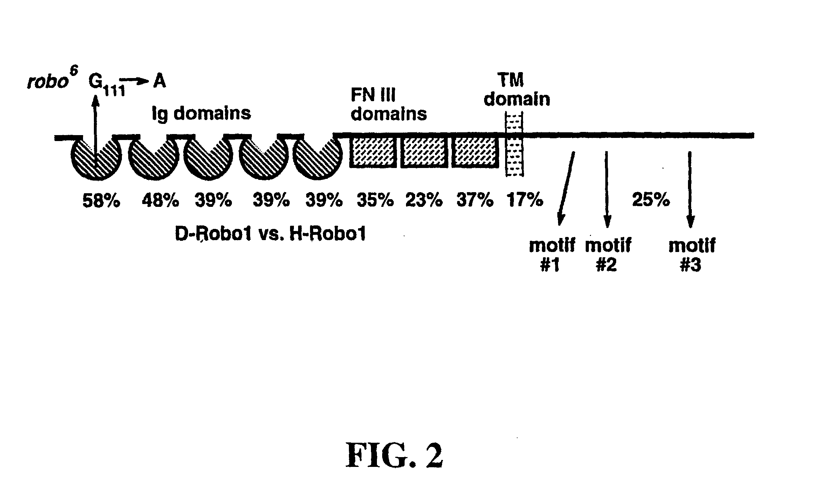

Figure 2 Structure of Robo Protein
   Schematic of the structure of Drosophila Robo protein. The position of the Immunoglobulin (Ig), fibronectin (FN) and transmembrane (TM) domains and the amino acid substitution in *robo*^{*6*} are shown. Percent amino acid identity between Drosophila Robo 1 and Human Robo 1 is indicated for each domain.

### DETAILED DESCRIPTION

The nucleotide sequences of exemplary natural cDNAs encoding drosophila 1, drosophila 2, C. elegans, human 1, human 2 and mouse 1 Robo polypeptides are shown as SEQ ID NOS:1, 3, 5, 7, 9 and 11, respectively, and the full conceptual translates are shown as SEQ ID NOS:2, 4, 6, 8, 10 and 12. The Robo polypeptides disclosed herein include incomplete translates of SEQ ID NOS:1, 3, 5, 7, 9 and 1 I and deletion mutants of SEQ ID NOS:2, 4, 6, 8, 10 and 12, which translates and deletion mutants have Robo-specific amino acid sequence, binding specificity or function. Preferred translates/deletion mutants comprise at least a 6, preferably at least an 8, more preferably at least a 32, most preferably at least a 64 residue domain of the translates. The deletion mutants comprise one or more structural/functional Robo immunoglobulin, fibronectin or cytoplasmic motif domains described herein. For example, soluble forms of the disclosed Robo polypeptides which comprise one or more Robo IG domains, and especially fusions of two or more Robo IG domains, particularly fusions of IG#1 and #2, provide competitive inhibitors of Robo-mediated signaling. Exemplary such deletion mutants and recombined deletion mutant fusions include human Robo 1 (SEQ ID NO:8) residues 1-67; 68-167; 168-259; 260-350; 351-451; 1-167; 1-259; 1-350; 1-451; 68-259; 1-67 joined to 168-259; and 1-67 joined to 260-451.

Other deletion mutants provide Robo-specific antigens and/or immunogens, especially when coupled to carrier proteins as described below. Generic Robo-specific peptides are readily apparent as conserved regions in the aligned Robo polypeptide sequences of Table 1.

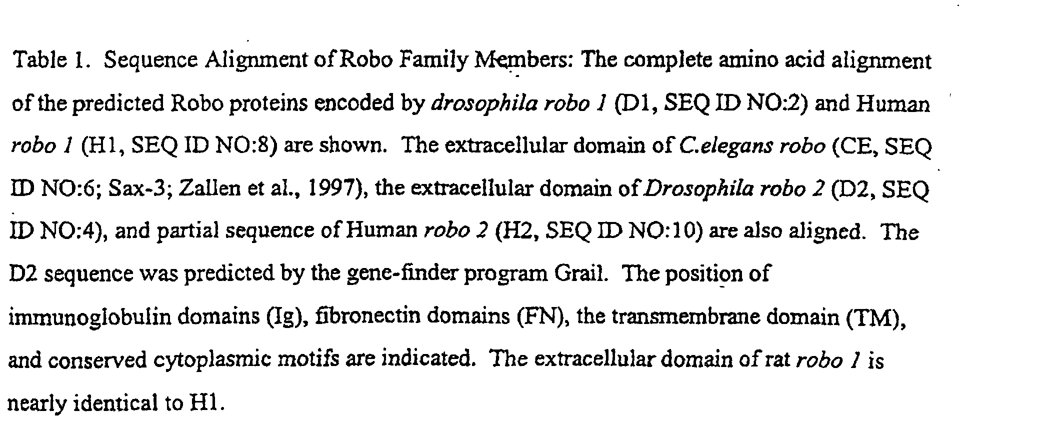

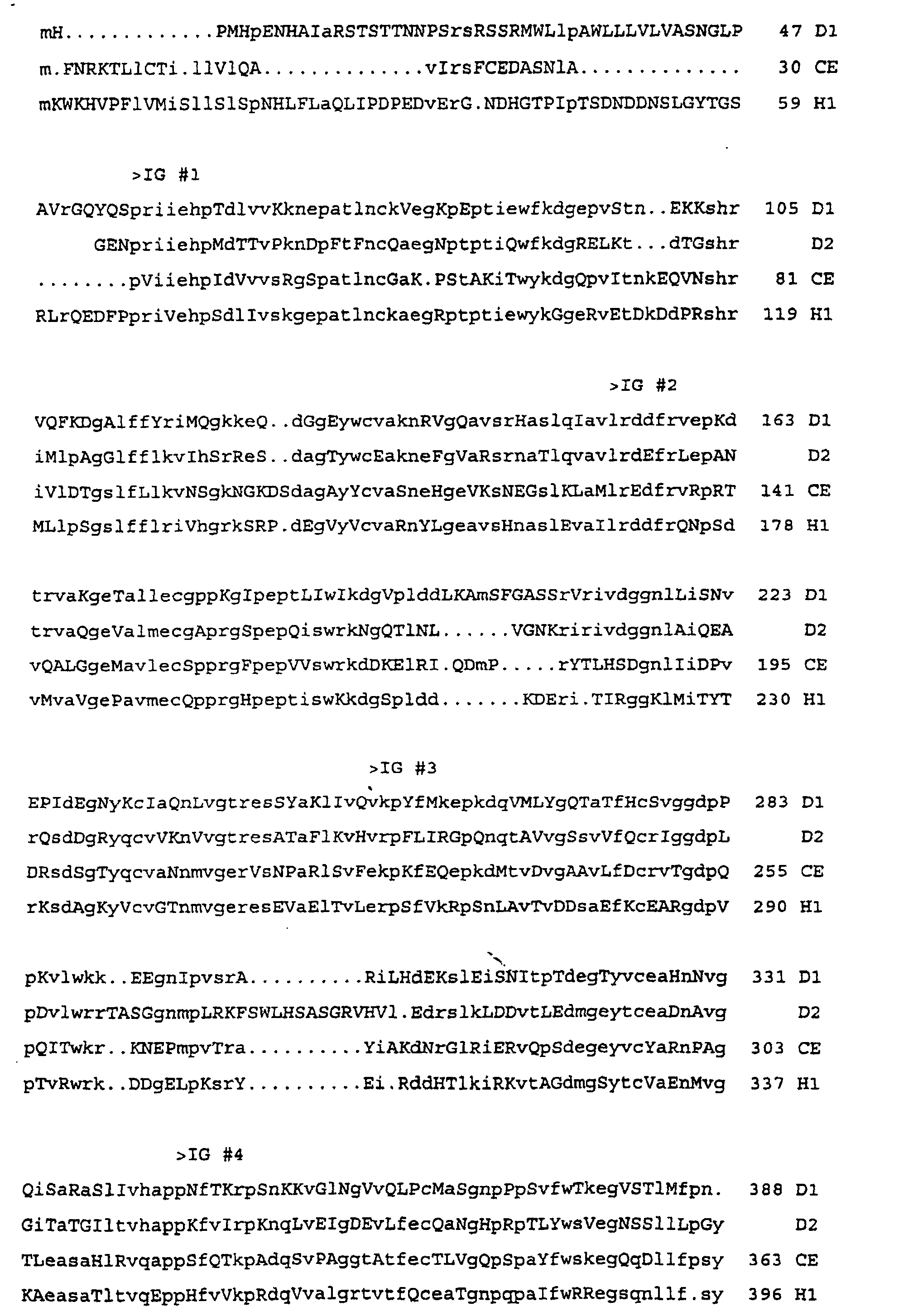

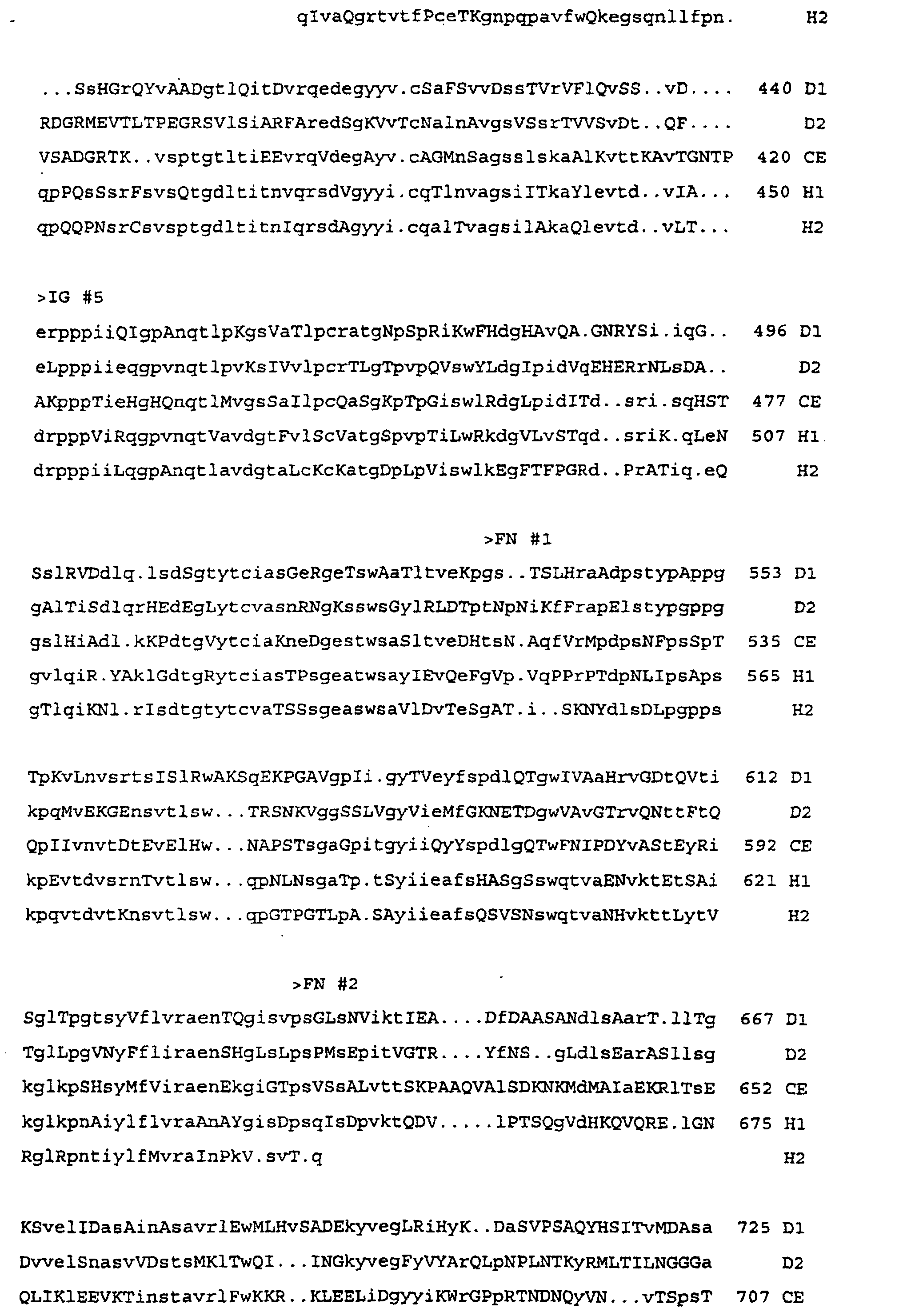

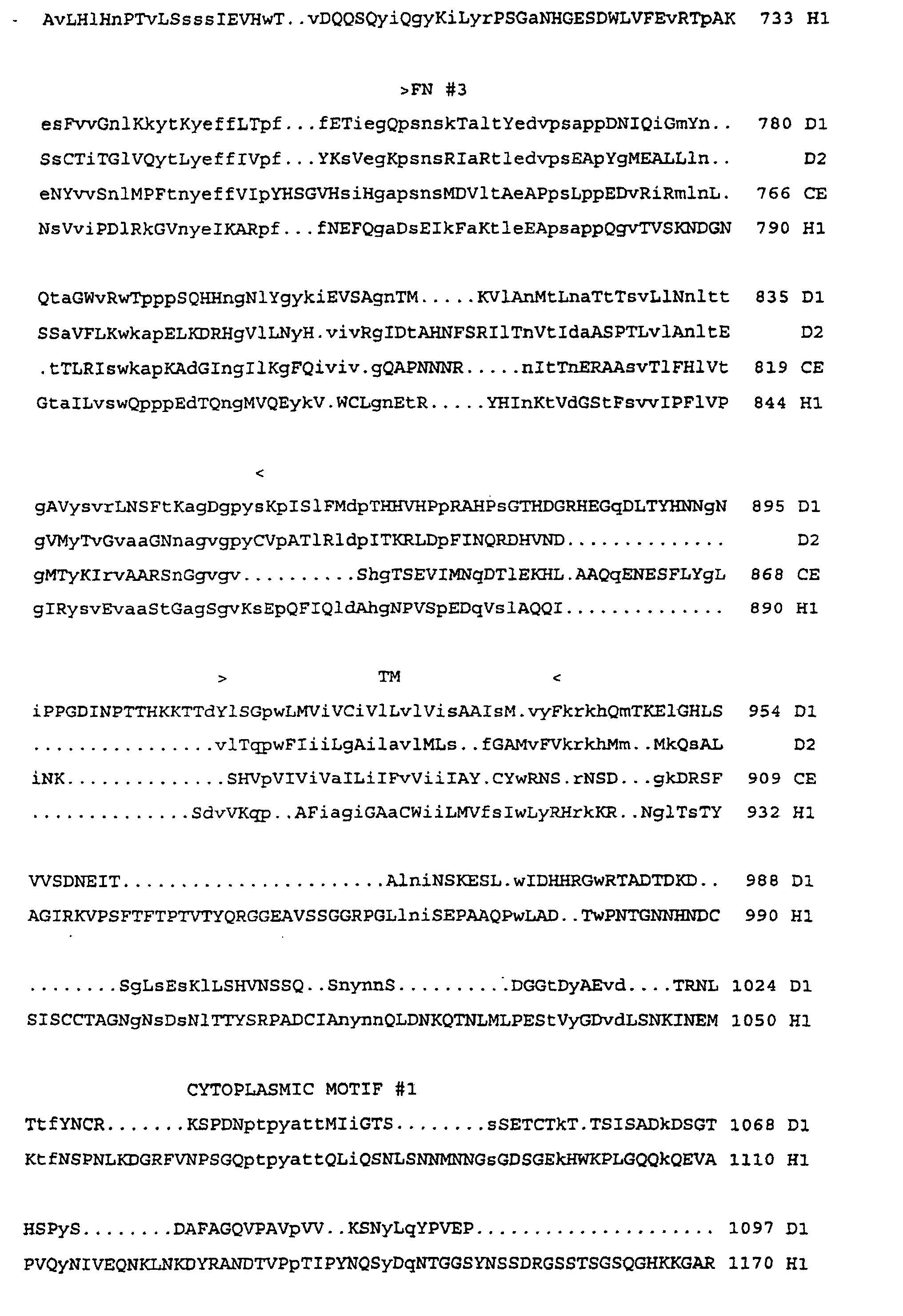

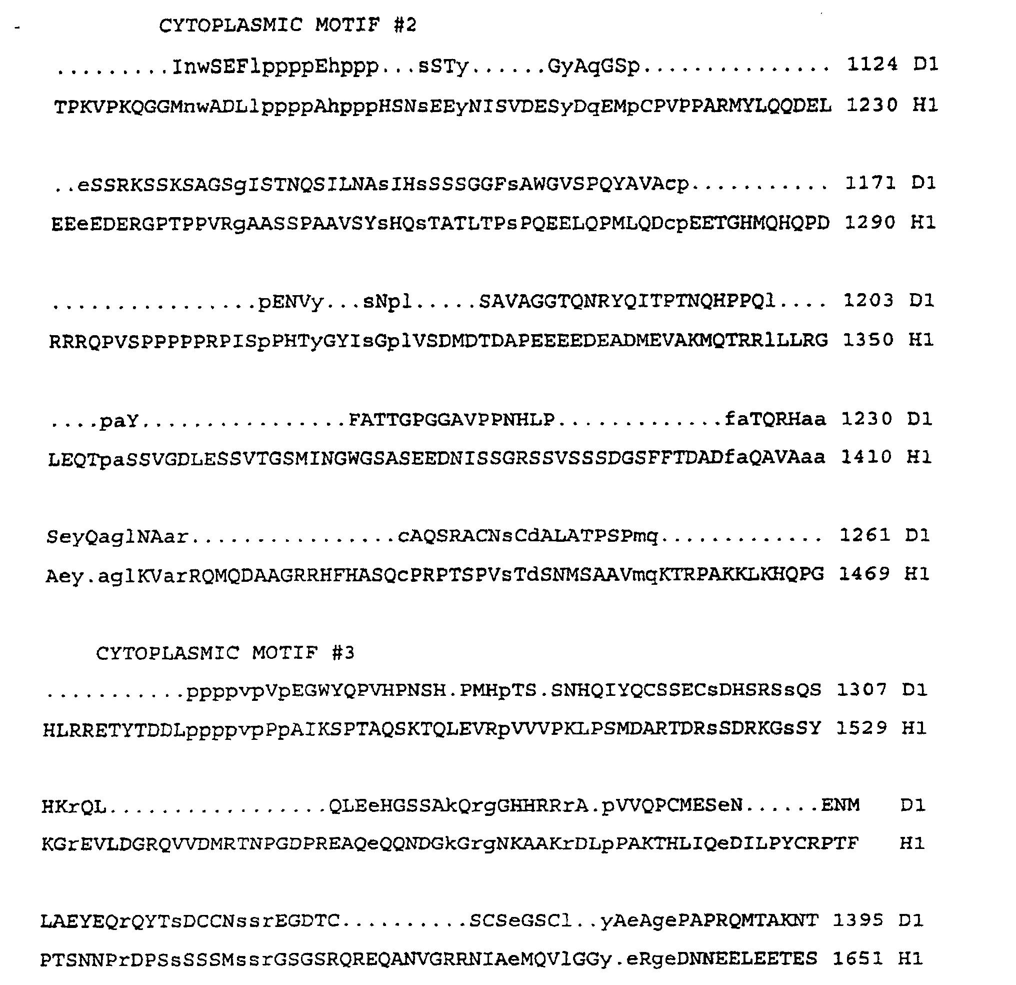

Exemplary such Robo specific immunogenic and/or antigenic peptides are shown in Table 2.

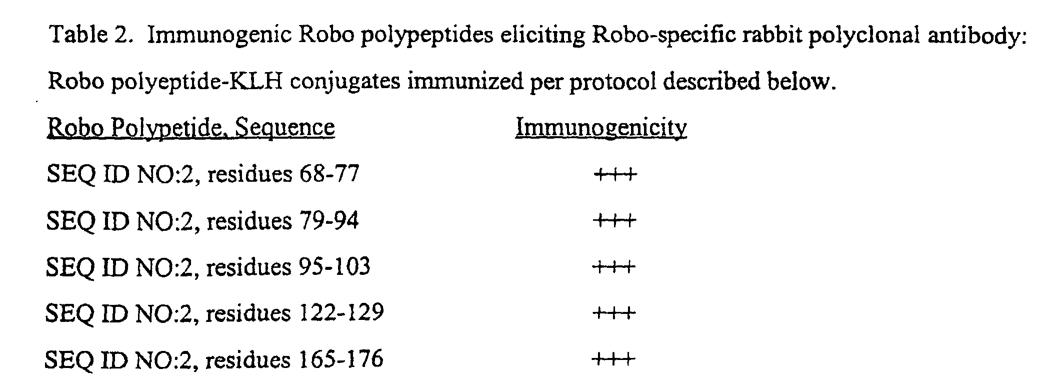

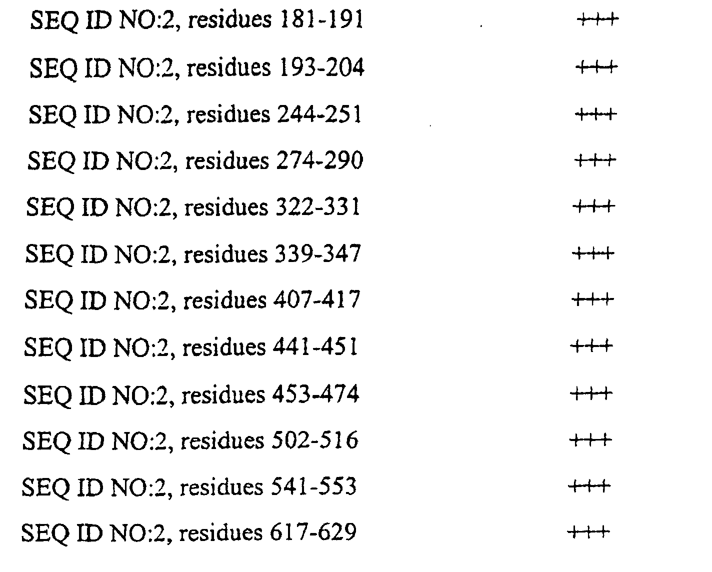

In addition, species-specific antigenic and/or immunogenic peptides are readily apparent as diverged extracellular or cytosolic regions in Table 1. Exemplary such human specific peptides are shown in Table 3.

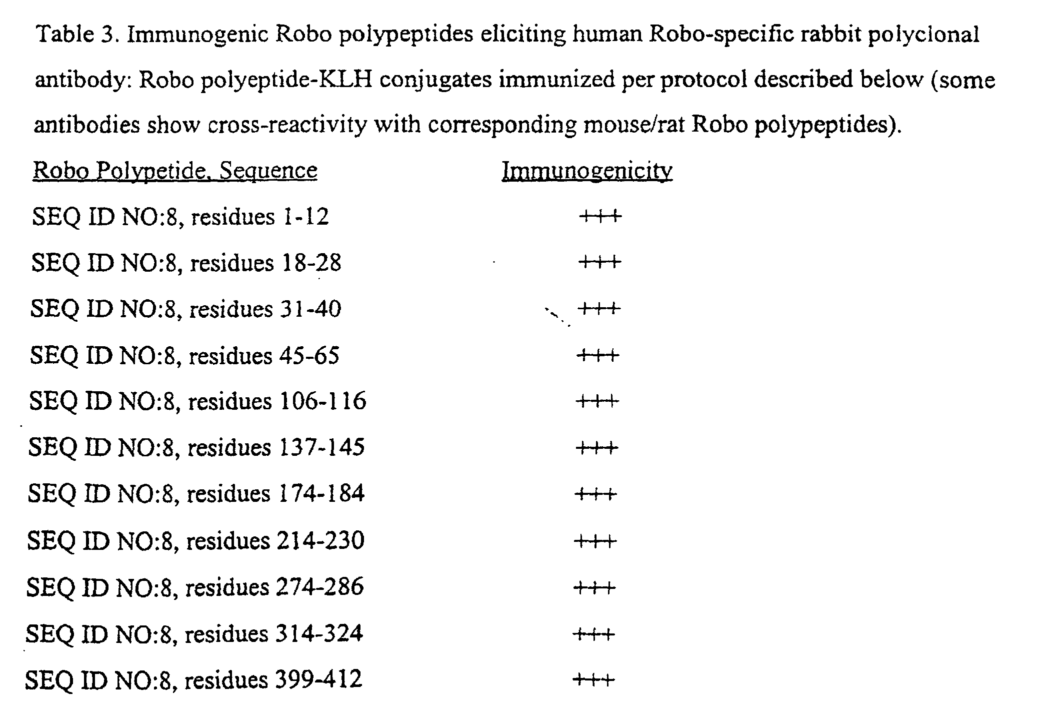

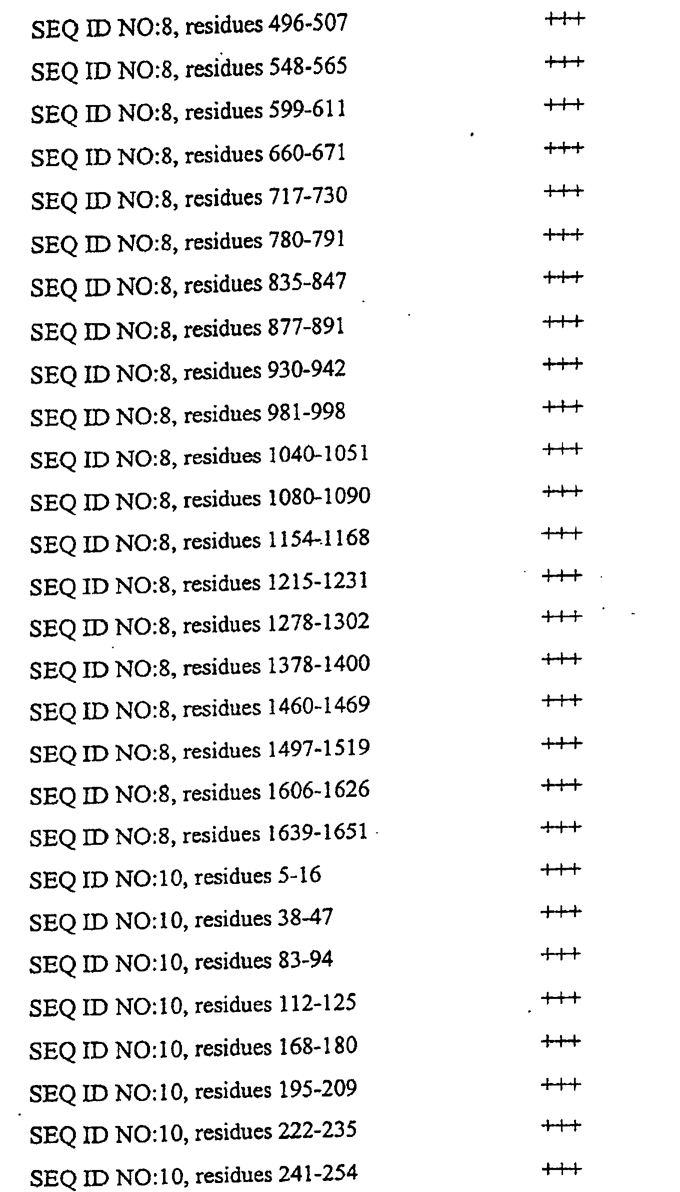

Expressed sequence tags EST;yu23d11, Accession #H77734 and EST;yq76e12, Accession #H52936, as well as peptides conceptually encoded thereby, are not within the scope of the present invention (Tables 4 and 5). The subject Robo polypeptides are not the corresponding regions of the disclosed natural human Robo I polypeptide, i.e. SEQ ID NO:8, residues 168-217 and SEQ ID NO:8, residues 1316-1485.

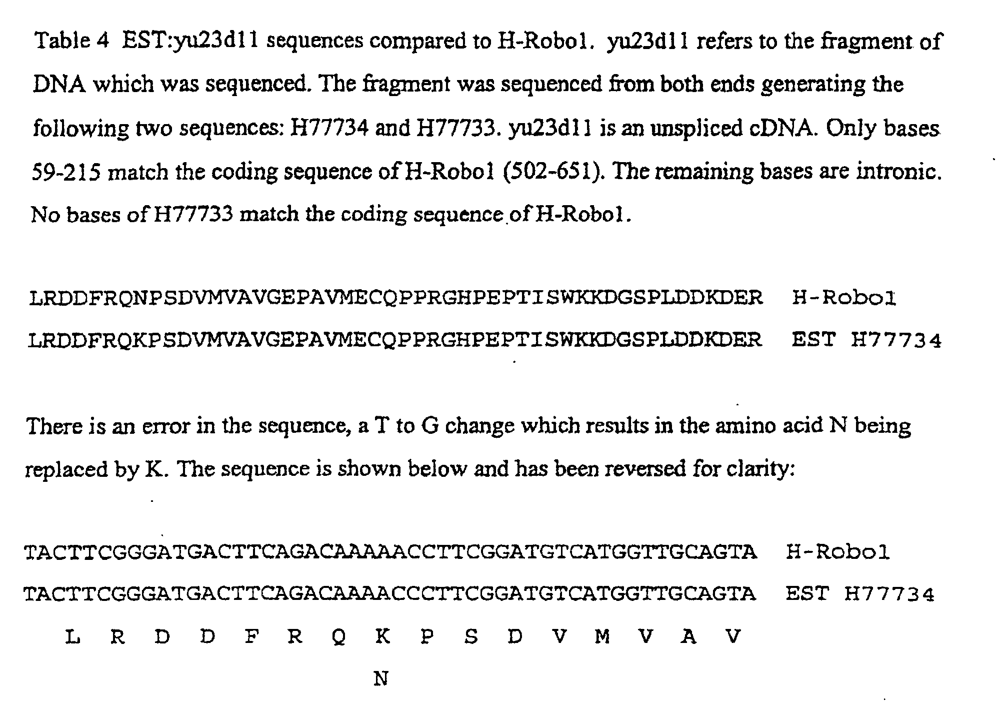

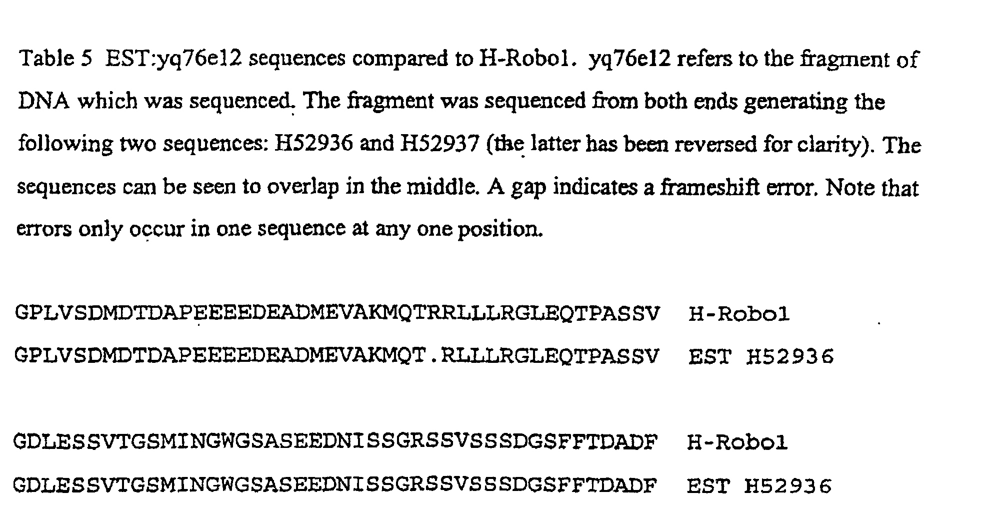

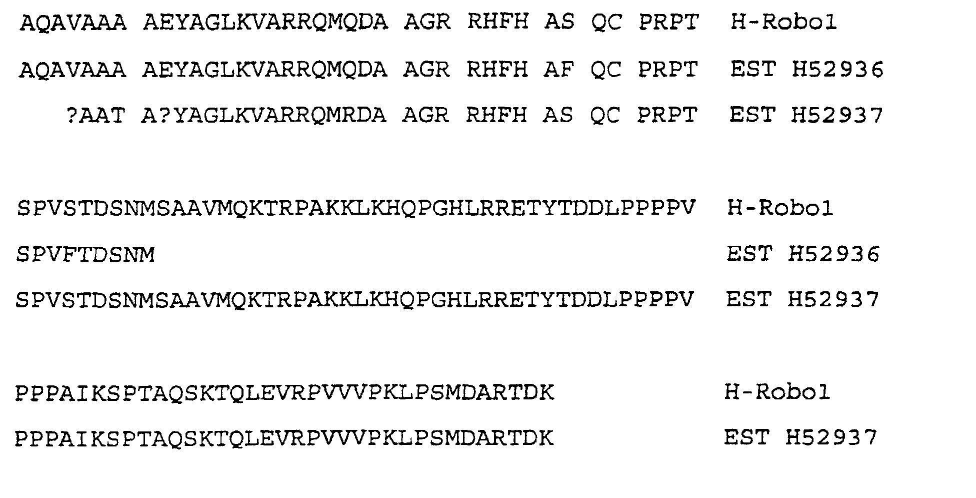

The subject domains provide Robo domain specific activity or function, such as Robo-specific cell, especially neuron modulating or modulating inhibitory activity, Robo-ligand-binding or binding inhibitory activity. Robo-specific activity or function may be determined by convenient *in vitro,* cell-based, or *in vivo* assays: e.g. *in vitro* binding assays, cell culture assays, in animals (e.g. gene therapy, transgenics, etc.), etc. Binding assays encompass any assay where the molecular interaction of a Robo polypeptide with a binding target is evaluated. The binding target may be a natural intracellular binding target, a Robo regulating protein or other regulator that directly modulates Robo activity or its localization; or non-natural binding target such as a specific immune protein such as an antibody, or a Robo specific agent such as those identified in screening assays such as described below. Robo-binding specificity may be assayed by binding equilibrium constants (usually at least about 10⁷M⁻¹, preferably at least about 10⁸ M⁻¹, more preferably at least about 10⁹ M⁻¹), by the ability of the subject polypeptide to function as negative mutants in Robo-expressing cells, to elicit Robo specific antibody in a heterologous host (e.g a rodent or rabbit), etc.

The claimed Robo polypeptides are isolated or pure: an "isolated" polypeptide is unaccompanied by at least some of the material with which it is associated in its natural state, preferably constituting at least about 0.5%, and more preferably at least about 5% by weight of the total polypeptide in a given sample and a pure polypeptide constitutes at least about 90%, and preferably at least about 99% by weight of the total polypeptide in a given sample. A polypeptide, as used herein, is a polymer of amino acids, generally at least 6 residues, preferably at least about 10 residues, more preferably at least about 25 residues, most preferably at least about 50 residues in length. The Robo polypeptides and polypeptide domains may be synthesized, produced by recombinant technology, or purified from mammalian, preferably human cells. A wide variety of molecular and biochemical methods are available for biochemical synthesis, molecular expression and purification of the subject compositions, see e.g. Molecular Cloning, A Laboratory Manual (Sambrook, *et al*. Cold Spring Harbor Laboratory), Current Protocols in Molecular Biology (Eds. Ausubel, *et al*., Greene Publ. Assoc., Wiley-Interscience, NY) or that are otherwise known in the art.

The disclosure provides binding agents specific to the claimed Robo polypeptides, including natural intracellular binding targets, etc., methods of identifying and making such agents, and their use in diagnosis, therapy and pharmaceutical development. For example, specific binding agents are useful in a variety of diagnostic and therapeutic applications, especially where pathology, wound repair incompetency or prognosis is associated with improper or undesirable axon outgrowth, orientation or inhibition thereof. Novel Robo-specific binding agents include Robo-specific receptors, such as somatically recombined polypeptide receptors like specific antibodies or T-cell antigen receptors (see, e.g Harlow and Lane (1988) Antibodies, A Laboratory Manual, Cold Spring Harbor Laboratory), natural intracellular binding agents identified with assays such as one-, two- and three-hybrid screens, non-natural intracellular binding agents identified in screens of chemical libraries such as described below, etc. Agents of particular interest modulate Robo function.

In a particular embodiment, the subject polypeptides are used to generate Robo- or human Robo-specific antibodies. For example, the Robo- and human Robo-specific peptides described above are covalently coupled to keyhole limpet antigen (KLH) and the conjugate is emulsified in Freunds complete adjuvant. Laboratory rabbits are immunized according to conventional protocol and bled. The presence of Robo-specific antibodies is assayed by solid phase immunosorbant assays using immobilized Robo polypeptides of SEQ ID NO:2, 4, 6, 8, 10 or 12. Human Robo-specific antibodies are characterized as uncross-reactive with non-human Robo polypeptides (SEQ ID NOS:2, 4, 6 and 12).

Accordingly, the disclosure provides methods for modulating cell function comprising the step of modulating Robo activity, e.g. by contacting the cell with a Robo inhibitor, e.g. inhibitory Robo deletion mutants, Robo-specific antibodies, etc. (supra). The target cell may reside in culture or in situ, i.e. within the natural host. The inhibitor may be provided in any convenient way, including by (i) intracellular expression from a recombinant nucleic acid or (ii) exogenous contacting of the cell. For many in situ applications, the compositions are added to a retained physiological fluid such as blood or synovial fluid. For CNS administration, a variety of techniques are available for promoting transfer of the therapeutic across the blood brain barrier including disruption by surgery or injection, drugs which transiently open adhesion contact between CNS vasculature endothelial cells, and compounds which facilitate translocation through such cells. Robo polypeptide inhibitors may also be amenable to direct injection or infusion, topical, intratracheal/nasal administration e.g. through aerosol, intraocularly, or within/on implants e.g. fibers e.g. collagen, osmotic pumps, grafts comprising appropriately transformed cells, etc. A particular method of administration involves coating, embedding or derivatizing fibers, such as collagen fibers, protein polymers, etc. with therapeutic proteins. Other useful approaches are described in Otto et al. (1989) J Neuroscience Research 22, 83-91 and Otto and Unsicker (1990) J Neuroscience 10, 1912-1921. Generally, the amount administered will be empirically determined, typically in the range of about 10 to 1000 µg/kg of the recipient and the concentration will generally be in the range of about 50 to 500 µg/ml in the dose administered. Other additives may be included, such as stabilizers, bactericides, etc. will be present in conventional amounts. For diagnostic uses, the inhibitors or other Robo binding agents are frequently labeled, such as with fluorescent, radioactive, chemiluminescent, or other easily detectable molecules, either conjugated directly to the binding agent or conjugated to a probe specific for the binding agent.

The amino acid sequences of the disclosed Robo polypeptides are used to back-translate Robo polypeptide-encoding nucleic acids optimized for selected expression systems (Holler et al. (1993) Gene 136, 323-328; Martin et al. (1995) Gene 154, 150-166) or used to generate degenerate oligonucleotide primers and probes for use in the isolation of natural Robo-encoding nucleic acid sequences ("GCG" software, Genetics Computer Group, Inc, Madison WI). Robo-encoding nucleic acids used in Robo-expression vectors and incorporated into recombinant host cells, e.g. for expression and screening, transgenic animals, e.g. for functional studies such as the efficacy of candidate drugs for disease associated with Robo-modulated cell function, etc.

The disclosure also provides nucleic acid hybridization probes (Tables 6, 7) and replication / amplification primers (Tables 7, 8) having a Robo cDNA specific sequence comprising SEQ ID NO:1, 3, 5, 7, 9 or 11 and sufficient to effect specific hybridization thereto (i.e. specifically hybridize with SEQ ID NO:1, 3, 5, 7, 9 or 11, respectively, in the presence of CDO cDNA.

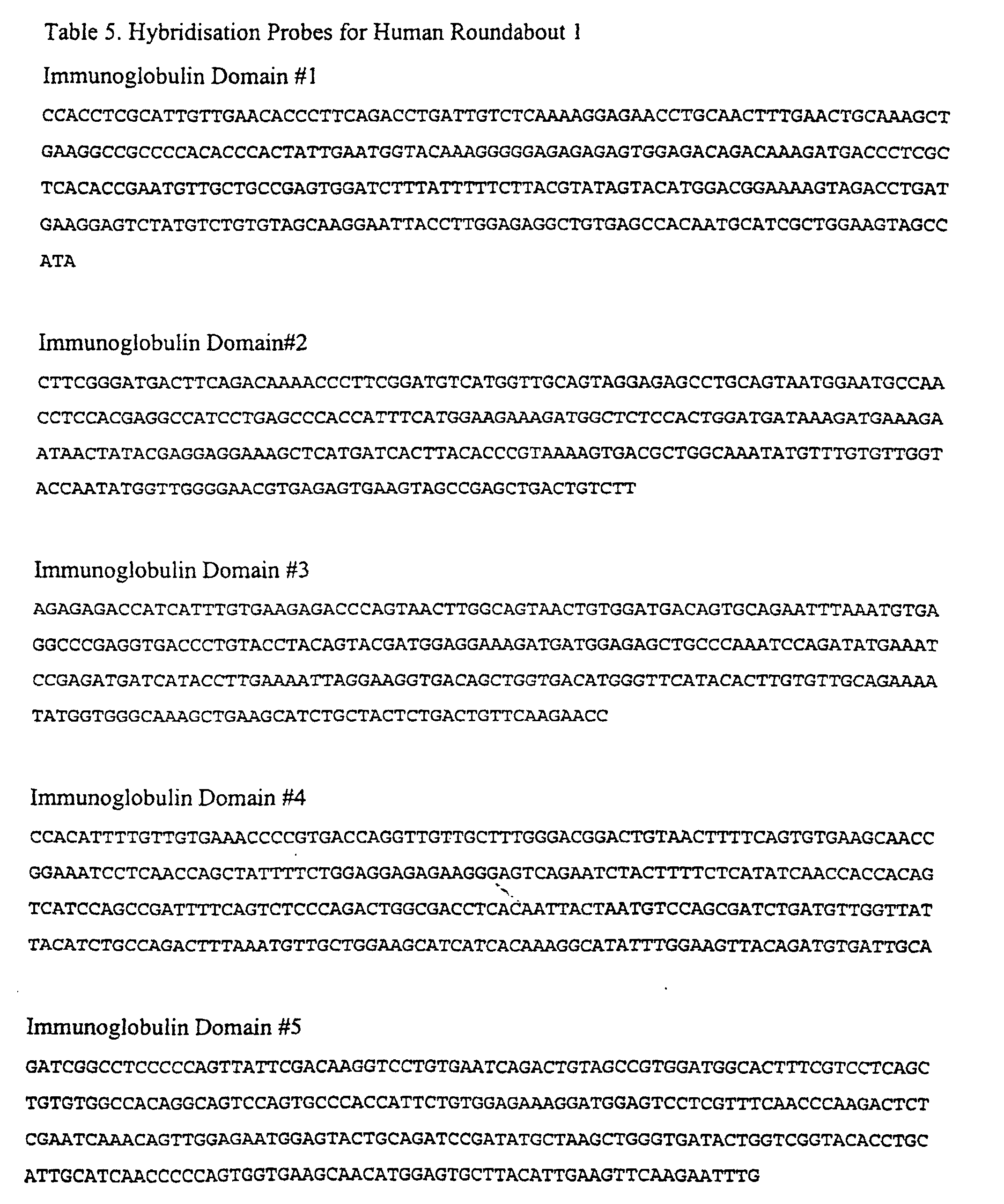

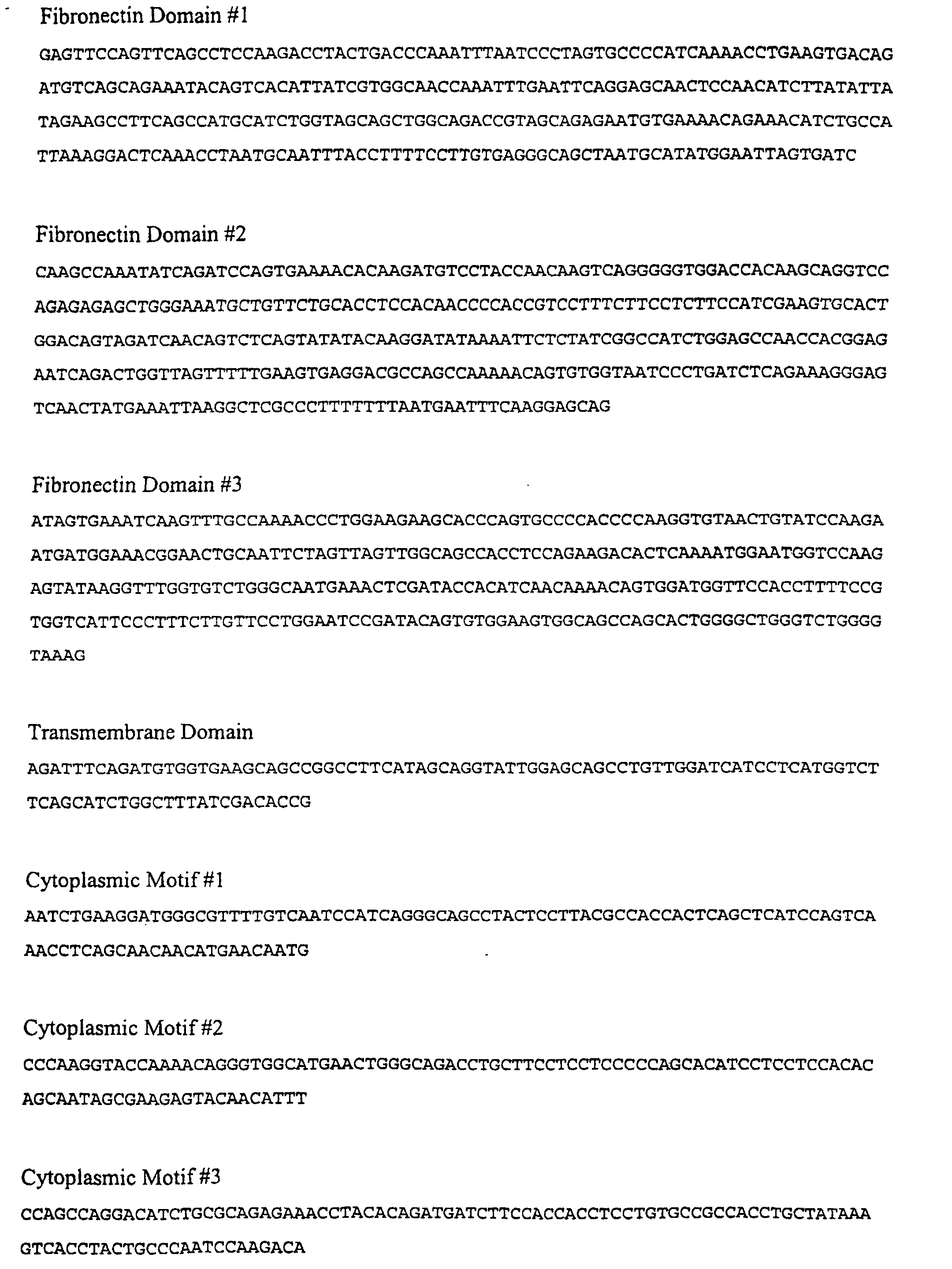

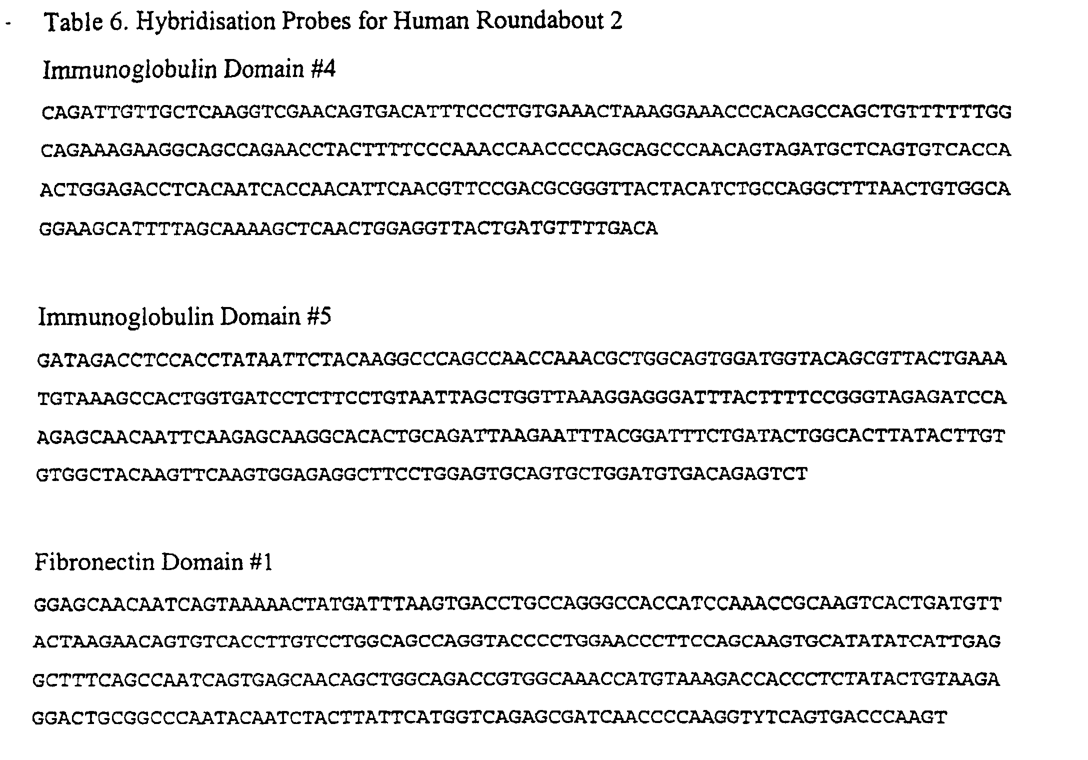

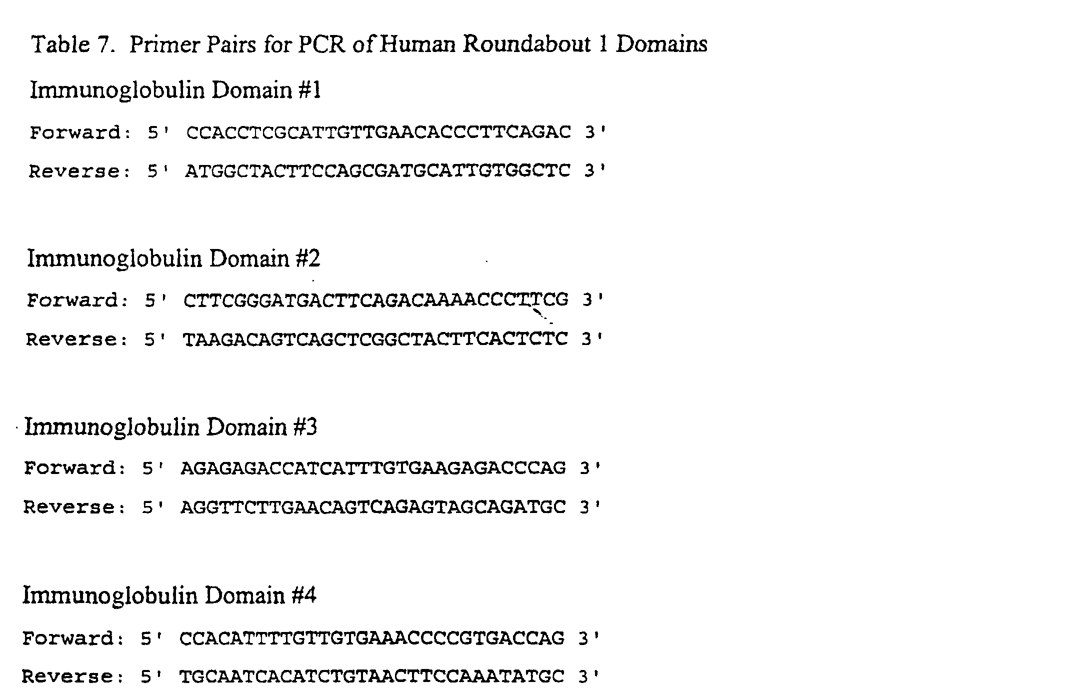

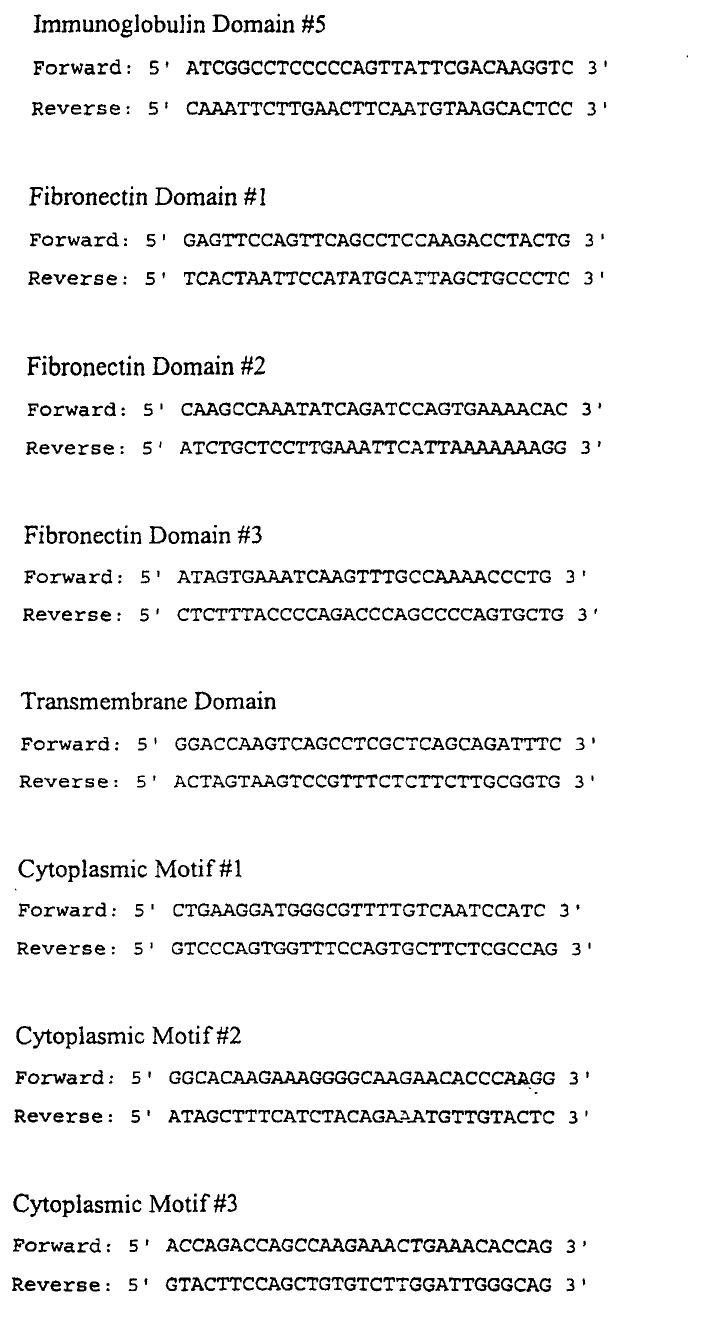

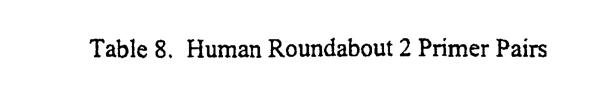

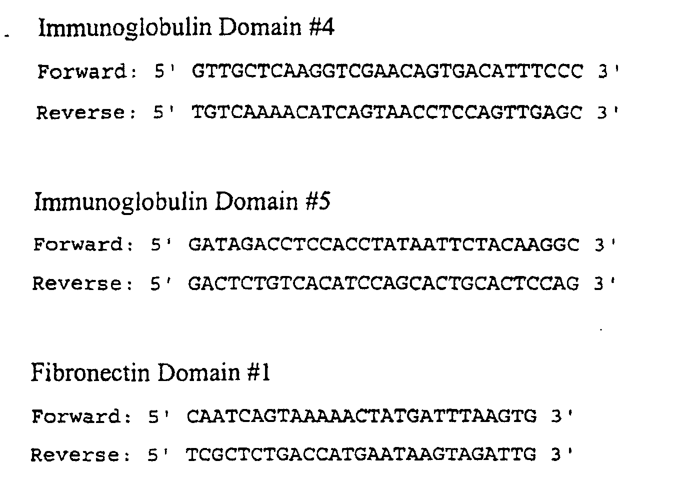

Such primers or probes are at least 12, preferably at least 24, more preferably at least 36 and most preferably at least 96 bases in length. Demonstrating specific hybridization generally requires stringent conditions, for example, hybridizing in a buffer comprising 30% formamide in 5 x SSPE (0.18 M NaCl, 0.01 M NaPO₄, pH7.7, 0.001 M EDTA) buffer at a temperature of 42°C and remaining bound when subject to washing at 42°C with 0.2 x SSPE; preferably hybridizing in a buffer comprising 50% formamide in 5 x SSPE buffer at a temperature of 42°C and remaining bound when subject to washing at 42°C with 0.2 x SSPE buffer at 42°C. Robo nucleic acids can also be distinguished using alignment algorithms, such as BLASTX (Altschul *et al*. (1990) Basic Local Alignment Search Tool, J Mol Biol 215, 403-410).

The subject nucleic acids are of synthetic/non-natural sequences and/or are isolated, i.e. unaccompanied by at least some of the material with which it is associated in its natural state, preferably constituting at least about 0.5%, preferably at least about 5% by weight of total nucleic acid present in a given fraction, and usually recombinant, meaning they comprise a non-natural sequence or a natural sequence joined to nucleotide(s) other than that which it is joined to on a natural chromosome. The subject recombinant nucleic acids comprising the nucleotide sequence of SEQ ID NO:1, 3, 5, 7, 9 or 11, or fragments thereof, contain such sequence or fragment at a terminus, immediately flanked by (i.e. contiguous with) a sequence other than that which it is joined to on a natural chromosome, or flanked by a native flanking region fewer than 10 kb, preferably fewer than 2 kb, more preferably fewer than 500 bp, which is at a terminus or is immediately flanked by a sequence other than that which it is joined to on a natural chromosome. While the nucleic acids are usually RNA or DNA, it is often advantageous to use nucleic acids comprising other bases or nucleotide analogs to provide modified stability, etc.

Expressed sequence tags EST;yu23d11, Accession #H77734 and EST;yq76e12, Accession #H52936, and deletion mutants thereof are not within the scope of the present invention. The subject Robo nucleic acids are not the corresponding regions of the disclosed natural human Robo I nucleic acids, i.e. SEQ ID NO:7, nucleotides 500-651 and SEQ ID NO:7, nucleotides 3945-4455.

| Table 10. Exemplary differences between H52936 and corresponding human Robo I sequences. | |
|---|---|
| (1) | At position 86, there is a T instead of an A. The new codon therefore reads TGA (Stop) instead of AGA (R). |
| (2) | There is a missing G at position 286-7, causing a frameshift. |
| (3) | There is an extra G at position 334, causing a frameshift. |
| (4) | There is an extra T at position 344, causing a frameshift. |
| (5) | There is an extra N at position 357, causing a frameshift. |
| (6) | There is a T instead of a C at 362. The new codon reads TTT (F) instead of TCT (S). |
| (7) | There is an extra T at position 364, causing a frameshift. |
| (8) | There is an extra N at position 370, causing a frameshift and a changed amino acid (the codon TTN is ambiguous). |
| (9) | There are two Ts at position 394 and 395 instead of a C, causing a frameshift and amino acid changes. |

| Table 11 . Exemplary differences between H52937 (reverse sequence) and corresponding human Robo I sequences. | |
|---|---|
| (1) | There are multiple errors in the first 30 bases. |
| (2) | At position 63, a G replaces an A. The new codon CGG codes for R instead of CAG for Q. |
| (3) | The EST ends by joining to part of the human glycophorin B gene (353-442) |

The subject nucleic acids find a wide variety of applications including use as translatable transcripts, hybridization probes, PCR primers, diagnostic nucleic acids, etc.; use in detecting the presence of Robo genes and gene transcripts and in detecting or amplifying nucleic acids encoding additional Robo homologs and structural analogs. In diagnosis, Robo hybridization probes find use in identifying wild-type and mutant Robo alleles in clinical and laboratory samples. Mutant alleles are used to generate allele-specific oligonucleotide (ASO) probes for high-throughput clinical diagnoses. In therapy, therapeutic Robo nucleic acids are used to modulate cellular expression or intracellular concentration or availability of active Robo.

The disclosure provides efficient methods of identifying agents, compounds or lead compounds for agents active at the level of a Robo modulatable cellular function. Generally, these screening methods involve assaying for compounds which modulate Robo interaction with a natural Robo binding target. A wide variety of assays for binding agents are provided including labeled *in vitro* protein-protein binding assays, immunoassays, cell based assays, etc. The methods are amenable to automated, cost-effective high throughput screening of chemical libraries for lead compounds. Identified reagents find use in the pharmaceutical industries for animal and human trials; for example, the reagents may be derivatized and rescreened in *in vitro* and *in vivo* assays to optimize activity and minimize toxicity for pharmaceutical development.

Cell and animal based neural guidance/repulsion assays are described in detail in the experimental section below. *In vitro* binding assays employ a mixture of components including a Robo polypeptide, which may be part of a fusion product with another peptide or polypeptide, e.g. a tag for detection or anchoring, etc. The assay mixtures comprise a natural intracellular Robo binding target. While native full-length binding targets may be used, it is frequently preferred to use portions (e.g. peptides) thereof so long as the portion provides binding affinity and avidity to the subject Robo polypeptide conveniently measurable in the assay. The assay mixture also comprises a candidate pharmacological agent. Candidate agents encompass numerous chemical classes, though typically they are organic compounds; preferably small organic compounds and are obtained from a wide variety of sources including libraries of synthetic or natural compounds. A variety of other reagents may also be included in the mixture. These include reagents like salts, buffers, neutral proteins, e.g. albumin, detergents, protease inhibitors, nuclease inhibitors, antimicrobial agents, etc. may be used.

The resultant mixture is incubated under conditions whereby, but for the presence of the candidate pharmacological agent, the Robo polypeptide specifically binds the cellular binding target, portion or analog with a reference binding affinity. The mixture components can be added in any order that provides for the requisite bindings and incubations may be performed at any temperature which facilitates optimal binding. Incubation periods are likewise selected for optimal binding but also minimized to facilitate rapid, high-throughput screening.

After incubation, the agent-biased binding between the Robo polypeptide and one or more binding targets is detected by any convenient way. Where at least one of the Robo or binding target polypeptide comprises a label, the label may provide for direct detection as radioactivity, luminescence, optical or electron density, etc. or indirect detection such as an epitope tag, etc. A variety of methods may be used to detect the label depending on the nature of the label and other assay components, e.g. through optical or electron density, radiative emissions, nonradiative energy transfers, etc. or indirectly detected with antibody conjugates, etc.

A difference in the binding affinity of the Robo polypeptide to the target in the absence of the agent as compared with the binding affinity in the presence of the agent indicates that the agent modulates the binding of the Robo polypeptide to the Robo binding target. For example, in the cell-based assay also described below, a difference in Robo-dependent modulation of axon outgrowth or orientation in the presence and absence of an agent indicates the agent modulates Robo function. A difference, as used herein, is statistically significant and preferably represents at least a 50%, more preferably at least a 90% difference.

The following experimental section and examples are offered by way of illustration and not by way of limitation.

### EXPERIMENTAL

Cloning of the *roundabout* Gene. The *robo*^{*1*} allele was mapped to the *plexus-brown* interval on the right arm of the second chromosome by recombination mapping; the numbers of recombinants suggested a map position very close to *plexus* at 58F/59A. One deficiency [*Df(2R)P*, which deletes 58E3/F1 through 60D14/E2] fails to complement *robo* mutations, two other deficiencies [*Df(2R)59AB* and *Df(2R)59AD*, which delete 59A1/3 through 59B1/2 and 59A1/3 through 59D1/4 respectively] do complement *robo*, and a duplication [*Dp(2;Y)bw*^{*+*}*Y*, which duplicates 58F1/59A2 through 60E3/F1] rescues *robo* mutations. This mapping places *robo* in the 58F/59A region.

We initiated chromosomal walks from P1 clones mapped to the region, beginning from the distal side using clone DS02204 and from the proximal side using clone DS05609. We used cosmid clones (Tamkun et al., 1992) to complete a walk of∼150 kb. We then looked for RFLPs in the recombinants between the multiple marked chromosome and the *robo* mutant chromosome. A 6.8kb EcoRI fragment from cosmid 106-5 identified a HindII RFLP on the mapping chromosome that was present on a single *robo* mutant recombinant line. This fragment identified a proximal limit for the location of *robo.* Further deficiencies in this region were then tested (Kerrebrock et al., 1995). Of these deficiencies, *Df(2R)X58-5* and *Df(2R)X58-12* remove *robo* while *Df(2R)X58-1* does not. *Df(2R)X58-12* fails to complement *Df(2R)59AB* yet complements *Df(2R)59AD* indicating that *Df(2R)59AB* extends further proximal; this proximal endpoint provides a distal limit for the location of *robo*. Probes from the walk were used to identify the breakpoints of these deficiencies (Figure 1A). *Df(2R)X58-1* breaks in a 9.6 kb EcoRI/BamHI fragment within cosmid GJ12, whereas *Df(2R) 59AB* breaks in a 8 kb BamHI/EcoRI fragment within cosmid 106-1435. This reduces the location of *robo* to a 75 kb region bounded by these restriction fragments. Hybridization of 0-16 hr poly-A⁺ embryonic Northern blots with cosmids GJ12, 106-12, and 106-1435 revealed at least five transcripts. Reverse Northern mapping identified the regions containing these transcripts (Figure 1A). These regions were used as probes to isolate cDNAs. Seven different cDNAs were isolated and analyzed by in situ hybridization. The expression pattern of five of these transcripts allowed us to tentatively discount them as encoding for *robo* since they were not expressed in the embryonic CNS at the appropriate stage. Of the two cDNAs remaining, 12-1 appeared by its size and expression the most likely candidate for *robo*. A 16 kb Xbal fragment including the 12-1 transcript and a region 5' to the transcript is capable of rescuing the *robo* mutant.

*roundabout* Encodes a Member of the Immunoglobulin Superfamily. We recovered and sequenced overlapping cDNA clones corresponding to the 12-1 transcription unit. A single long open reading frame (ORF) that encodes 1395 amino acids was identified (D1 in Table 1). Conceptual translation of the ORF reveals the Robo protein to be a member of the Ig superfamily; Robo's ectodomain contains five immunoglobulin (Ig)-like repeats followed by three fibronectin (Fn) type-III repeats. The predicted ORF also contains a transmembrane domain and a large 457 amino acid (a.a.) cytoplasmic domain. Hydropathy analysis of the Robo sequence indicates a single membrane spanning domain of 25 a.a. (Kyte and Doolittle, 1982) plus a signal sequence with a predicted cleavage site between G51 and Q52 (Nielsen et al 1997).

We identify the 12-1 transcript as encoding *robo* based on several criteria. First, the embryonic *robo* phenotype can be rescued by the 16 kb XbaI genomic fragment containing this cDNA; no other transcripts are contained in this 16 kb XbaI fragment. Second, we identified a CfoI RFLP associated with the allele *robo*^{*6*}. This polymorphism is due to a change of nucleotide 332 of the ORF from G to A, which results in a change of Gly₁₁₁ to Asp. Glyl 11 is in the first Ig domain (Figure 2), and is conserved in all Robo homologues identified. The change is specific to the allele *robo*^{*6*} and is not seen in the parental chromosome or in any of the other seven alleles, all of which were generated from the same parental genotype. Third, the production of antibodies (below) which recognize the Robo protein reveals that the alleles *robo*^{*1*}*, robo*^{*2*}*, robo*^{*3*}*, robo*^{*4*} and *robo*^{*5*} do not produce Robo protein (Table 12).

**Table 12.**

| *robo* Mutant Alleles | | |
|---|---|---|
| Allele | Synonym | Class |
| *robo*^{*1*} | GA285 | Protein null |
| *robo*^{*2*} | GA1112 | Protein null |
| *robo*^{*3*} | Z14 | Protein null |
| *robo*^{*4*} | Z570 | Protein null |
| *robo*^{*5*} | Z1772 | Protein null |
| *robo*^{*6*} | Z1757 | Protein positive; Gly₁₁₁ to Asp |
| *robo*^{*7*} | 22130 | Reduced protein levels |
| *robo*^{*8*} | 23127 | Protein positive |

All alleles were generated by EMS mutagenesis of *FasIII* null chromosomes. Each of these alleles appear to represent a complete, or near complete, loss-of-function phenotype for *robo,* since the mutant phenotype observed when these alleles are placed over a chromosome deficient for the *robo* locus [Df(2R) X58-5] is indistinguishable from the homozygous allele.
Finally, transgenic neural expression of *robo* rescues the midline crossing phenotype of *robo* mutants (see below).

Developmental Northern blot analysis using both cDNA and genomic probes suggests that *robo* is encoded by a single transcript of∼7500 bp. We sequenced genomic DNA and identified 17 introns within the sequence of which 14 are only 50-75 bp in length plus three introns of 843 bp, 236 bp, and 110 bp (Figure 1B). The precise start point of the transcript has not been determined.

A Family of Evolutionarily Conserved Robo-like Proteins. The presence of five Ig and three Fn domains, a transmembrane domain, and a long (452 a.a.) cytoplasmic region indicates that Robo may be a receptor and signaling molecule. The netrin receptor DCC/Frazzled/UNC-40 has a related domain structure, with 6 Ig and 4 Fn domains and a similarly long cytoplasmic region (Keino-Masu et al., 1996; Chan et al., 1996; Kolodziej et al., 1996). The only currently known protein with a "5 + 3" organization is CDO (Kang et al., 1997). However, CDO is only distantly related to Robo (15-33% a.a. identity between corresponding Ig and FN domains).

We identified other "5 + 3" proteins in vertebrates whose amino acid identity exceeds that of CDO and represent Robo homologues. A human expressed sequence tag (EST; yu23d11, Accession #H77734) shows high homology to the second Ig domain of *robo* and was used to probe a human fetal brain cDNA library (Stratagene). The clones recovered correspond to a human gene with five Ig and three Fn domains (Figure 2). Exemplary functional Robo domains are listed in Tables 13-17 (the corresponding encoding nucleic acids are readily discernable from the corresponding nucleic acid sequences of Sequence Listing).

**Table 13.**

| Exemplary domains of human Robo 1, by amino acid sequence positions | |
|---|---|
| Signal sequence | 6-21 |
| First Immunoglobulin domain | 68-167 |
| Second Immunoglobulin domain | 168-258 |
| Third Immunoglobulin domain | 259-350 |
| Fourth Immunoglobulin domain | 351-450 |
| Fifth Immunoglobulin domain | 451-546 |
| First Fibronectin domain | 547-644 |
| Second Fibronectin domain | 645-761 |
| Third Fibronectin domain | 762-862 |
| Transmembrane domain | 896-917 |
| Cytoplasmic motif #1 | 1070-1079 |
| Cytoplasmic motif #2 | 1181-1195 |
| Cytoplasmic motif #3 | 1481-1488 |

**Table 14.**

| Exemplary domains of human Robo II, by amino acid sequence positions | |
|---|---|
| Fourth Immunoglobulin domain | 1-91 |
| Fifth Immunoglobulin domain | 92-185 |
| First Fibronectin domain | 186-282 |

**Table 15.**

| Exemplary domains of drosophila Robo 1, by amino acid sequence positions | |
|---|---|
| Signal sequence | 30-46 |
| First Immunoglobulin domain | 56-152 |
| Second Immunoglobulin domain | 153-251 |
| Third Immunoglobulin domain | 252-344 |
| Fourth Immunoglobulin domain | 345-440 |
| Fifth Immunoglobulin domain | 441-535 |
| First Fibronectin domain | 536-635 |
| Second Fibronectin domain | 636-753 |
| Third Fibronectin domain | 754-854 |
| Transmembrane domain | 915-938 |
| Cytoplasmic motif #1 | 1037-1046 |
| Cytoplasmic motif #2 | 1098-1119 |
| Cytoplasmic motif #3 | 1262-1269 |

**Table 16.**

| Exemplary domains of drosophila Robo II, by amino acid sequence positions | |
|---|---|
| Immunoglobulin domain #1 | 4-99 |
| Immunoglobulin domain #2 | 100-192 |
| Immunoglobulin domain #3 | 193-296 |
| Immunoglobulin domain #4 | 297-396 |
| Immunoglobulin domain #5 | 397-494 |
| Fibronectin domain #1 | 495-595 |
| Fibronectin domain #2 | 596-770 |
| Fibronectin domain #3 | 771-877 |
| Transmembrane domain | 906-929 |
| Conserved cytoplasmic motif#1 | 1075-1084 |

**Table 17.**

| Exemplary domains of C. elegans Robo 1, by amino acid sequence positions | |
|---|---|
| First Immunoglobulin domain | 30-129 |
| Second Immunoglobulin domain | 130-223 |
| Third Immunoglobulin domain | 224-315 |
| Fourth Immunoglobulin domain | 316-453 |
| Fifth Immunoglobulin domain | 454-543 |
| First Fibronectin domain | 544-643 |
| Second Fibronectin domain | 644-766 |
| Third Fibronectin domain | 767-865 |
| Transmembrane domain | 900-922 |
| Cytoplasmic motif #1 | 1036-1045 |
| Cytoplasmic motif #2 | 1153-1163 |
| Cytoplasmic motif #3 | 1065-1074 |

The homology is particularly high in the first two Ig domains (58% and 48% a.a. identity respectively, compared to 26% and 30% for the same two Ig domains between D-Robo1 and CDO) and together with the overall identity throughout the extracellular region and the presence of three conserved cytoplasmic motifs has led us to designate this as the human *roundabout 1* gene (*H-robo1*). Database searching reveals a nucleotide sequence corresponding to *H-robo1* in the database, *DUTT1*, which differs in the signal sequence suggesting alternative splicing, a 9 bp insertion and seven single base pair changes. Five ESTs (see Experimental Procedures) show high sequence similarity to the cytoplasmic domain of *H-robo1*. Sequencing of cDNAs isolated using one of these ESTs as a probe confirmed a second human *roundabout* gene (*H-robo2*).

Degenerate PCR primers based on conserved sequences between *H-robo1* and *D-robo1* were used to isolate a PCR fragment from a rat embryonic E13 brain cDNA library. The fragment was used to probe an E 13 spinal cord cDNA library, resulting in the isolation of a full length Rat *robo* gene (*R-robo1*). The predicted protein shows high sequence identitiy (>95%) with *H-robo1* over the entire length. The 5' sequences of different *R-robo1* cDNA clones indicates that this gene is alternatively spliced in a similar fashion to *H-robo1*/*DUTTI*. We used a similar approach to isolate cDNA clones for *R-robo2*, which is highly homologous to *H-robo2*.

The mouse EST vi92e02 is highly homologous to the cytoplasmic portion of *H-robo1.* The *C. elegans Sax-3* gene is also *a robo* homologue (Table 1; Zallen et al., 1997). A second Drosophila *robo* gene (*D-robo2*) is also predicted from analysis of genomic sequence in the public database. Taken together these data indicate that Robo is the founding member of a new subfamily of Ig superfamily proteins with at least one member in nematode, two in Drosophila, two in rat, and two in human.

The alignment of the Robo family proteins reveals that the first and second Ig domains are the most highly conserved portion of the extracellular domain. The cytoplasmic domains are highly divergent except for the presence of three highly conserved motifs (Table 18).

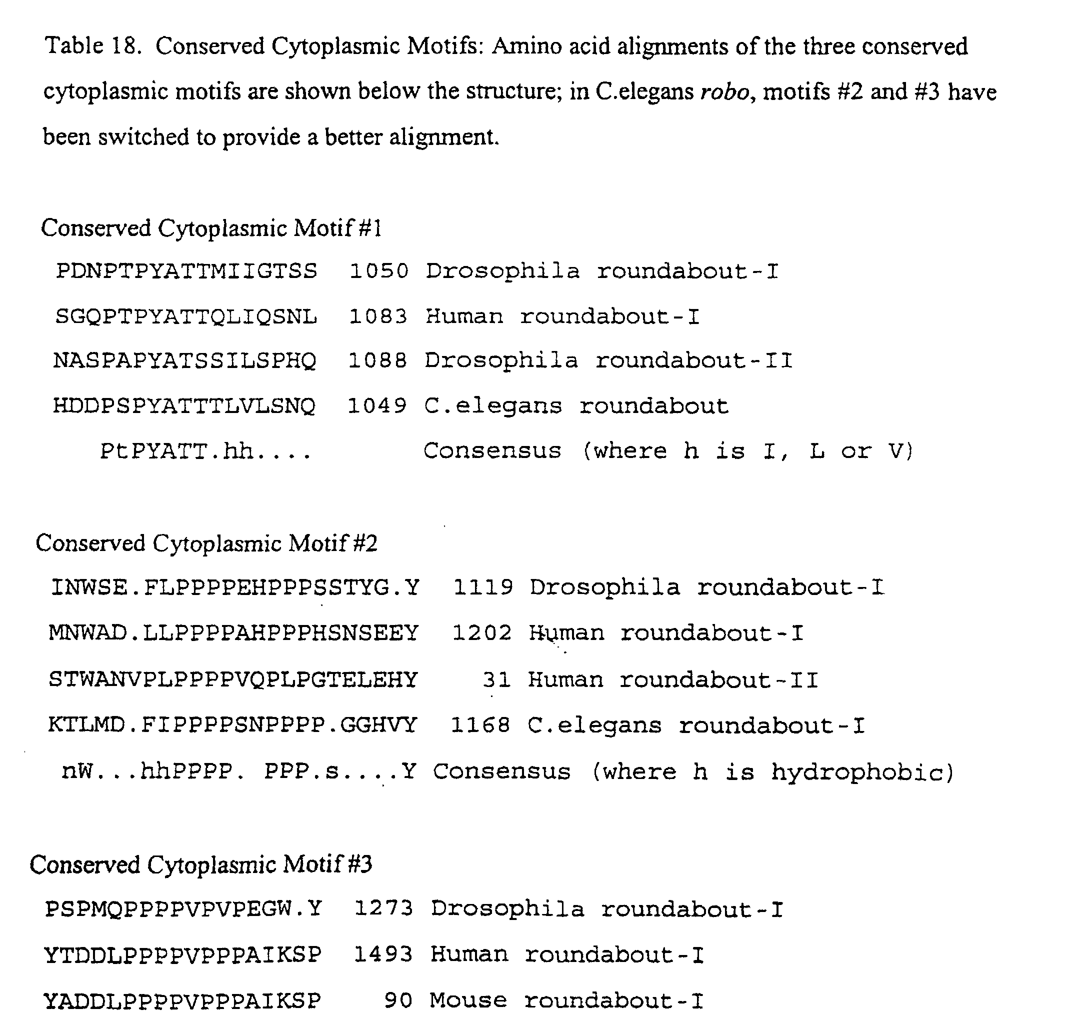

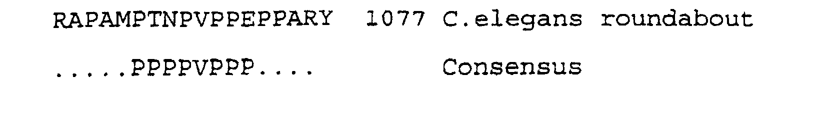

The consensus for the first motif is PtPYATTxhh, where x is any amino acid and h is I, L, or V. The presence of a tyrosine in the center of the motif indicates a site for phosphorylation. The other two motifs consist of runs of prolines separated by one or two amino acids and are reminiscent of binding sites for SH3 domains. In particular, the LPPP sequence in motif #2 provides a good binding site for the Drosophila Enabled protein or its mammalian homologue Mena (Niebuhr et al., 1997). All three of these conserved sites can function as binding sites for domains (e.g. SH3 domains) of linker/adapter proteins functioning in Robo-mediated signal transduction.

Robo is Regionally Expressed on Longitudinal Axons in the Drosophila Embryo. In order to determine the role that *robo* might play in regulating axon crossing behavior, we examined the *robo* expression pattern in the embryonic CNS. The in situ hybridization pattern *of robo* mRNA in Drosophila shows it to have elevated and widespread expression in the CNS. We raised a monoclonal antibody (MAb 13C9) against part of the extracellular portion (amino acids 404-725) of the protein to visualize Robo expression. Robo is first seen in the embryo weakly expressed in lateral stripes during germband extension. At the onset of germband retraction, Robo expression is observed in the neuroectoderm. By the end of stage 12, as the growth cones first extend, Robo is seen on growth cones which project ipsilaterally, including pCC, aCC, MP1, dMP2, and vMP2. Strikingly, little or no Robo expression is observed on commissural growth cones as they extend towards and across the midline. However, as these growth cones turn to project longitudinally, their level of Robo expression dramatically increases. Robo is expressed at high levels on all longitudinally-projecting growth cones and axons. In contrast, Robo is expressed at nearly undetectable levels on commissural axons. This is striking since ∼90% of axons in the longitudinal tracts also have axon segments crossing in one of the commissures. Thus, Robo expression is regionally restricted. Robo expression is also seen at a low level throughout the epidermis and at a higher level at muscle attachment sites. In stage 16-17 embryos, faint Robo staining can be seen in the commissures but at levels much lower than observed in the longitudinal tracts.

Immunoelectron Microscopy of Robo. We used immunoelectron microscopy to examine Robo localization at higher resolution. In stage 13 embryos, Robo is expressed at higher levels on growth cones and filopodia in the longitudinal tracts than on the longitudinal axons themselves. This localization is consistent with the model that Robo functions as a guidance receptor. The increased sensitivity of immunoelectron microscopy reveals the presence of very low levels of Robo protein on the surface of commissural axons. In addition, Robo-positive vesicles can be seen inside the commissural axons, possibly representing transport of Robo to the growth cone. Finally, by reconstructing the path of single axons by use of serial sections, we confirm that Robo expression is greatly up-regulated after individual axons turn from the commissure into a longitudinal tract. The expression of Robo on non-crossing and post-crossing axons and its higher level of expression on growth cones and its filopodia, provide a model where Robo functions as an axon guidance receptor for a repulsive midline cue.

Transgenic Expression of Robo. We hypothesized that if Robo is indeed a growth cone receptor for a midline repellent, then pan-neural expression of Robo protein during the early stages of axon outgrowth might lead to a *robo* gain-of-function phenotype similar to the *comm* loss-of-function and opposite of the *robo* loss-of-function. To test this hypothesis, we cloned a *robo* cDNA containing the complete ORF but lacking most of its untranslated regions (UTRs) downstream of the UAS promoter in the pUAST vector and generated transgenic flies for use in the GAL4 system (Brand and Perrimon, 1993). Expression of *robo* in all neurons was achieved by crossing the *UAS-robo* flies to either the *elav-GAL4* or *scabrous-GAL4* lines.

Surprisingly, pan-neural expression of *robo* mRNA did not produce a strong axon scaffold phenotype as assayed with MAb BP102. Staining with anti-Fas II (MAb 1D4) revealed subtle fasciculation defects, but overall the axon scaffold looked quite normal. An insight into why we failed to observe a stronger *robo* ectopic expression phenotype was provided by staining these embryos with the anti-Robo MAb. Interestingly, the Robo protein, although expressed at higher levels than in wild type, remains restricted as in wild type, i.e., high levels of expression on the longitudinal portions of axons and very low levels on the commissures. This result indicates that there must be strong regulation of Robo expression, probably post-translational, that assures its localization to longitudinal axon segments. Such a mechanism could operate by the regulation of protein translation, transport, insertion, internalization and/or stability.

We used these transgenic flies to rescue *robo* mutants. Expression of *robo* by the *elav* *GAL4* line in both *robo*^{*3*} and *robo*^{*5*} homozygotes rescued the midline crossing of Fas II positive axons including pCC and other identified neurons.

Robo Appears to Function in a Cell Autonomous Fashion. To test whether Robo can function in a cell autonomous fashion, we used the *UAS-robo* transgene with the *ftz*_{*ng*}*-GAL4* line (Lin et al., 1994). The *ftz*_{*ng*}*-GAL4* line expresses in a subset of CNS neurons, including many of the earliest neurons to be affected by the *robo* mutation such as pCC, vMP2, dMP2, and MP1. Expression of *robo* by the *ftz*_{*ng*}*-GAL4* line is sufficient to rescue these identified neurons in the *robo* mutant: pCC, which in robo mutants heads towards and crosses the midline, in these rescued embryos now projects ipsilaterally and does not cross the midline. When the same embryos were stained with the anti-robo MAb 13C9, we observed that all Robo-positive axons did not cross the midline. The *ftz*_{*ng*}*-GAL4* line drives expression in many of the axons in the pCC pathway (Lin et al., 1994), a medial longitudinal fascicle. In *robo* mutants, this axon fascicle freely crosses and circles the midline, joining with its contralateral pathway. When rescued by the *ftz*_{*ng*}*-GAL4* line driving *UAS-robo,* this pathway now largely remains on its own side of the midline, even though occasionally a few axons cross the midline. These experiments support the notion that Robo can function in a cell autonomous fashion.

Expression of Mammalian *robo1* in the Rat Spinal Cord. The isolation of several vertebrate Robo homologues suggests that Robo may play a similar role in orchestrating midline crossing in the vertebrate nervous system as it does in Drosophila. In the vertebrate spinal cord, the ventral midline is comprised of a unique group of cells called the floor plate (for review, Colamarino and Tessier-Lavigne, 1995). As in the Drosophila nervous system, the vertebrate spinal cord contains both crossing and non-crossing axons. Spinal commissural neurons are born in the dorsal half of the spinal cord; commissural axons project to and cross the floor plate before turning longitudinally in a rostral direction. In contrast, the axons of two other classes of neurons, dorsal association neurons and ventral motor neurons, do not cross the floor plate (Altman and Bayer, 1984).

To address the possibility that Robo may play a role in organizing the projections of these spinal neurons, we examined the expression of rat *robo1* by RNA in situ hybridization. A rat *robo1* riboprobe spanning the first three Ig domains was hybridized to transverse sections of E13 rat spinal cord. At E13, when many commissural axons will have already extended across the floor plate (Altman and Bayer, 1984), rat *robo1* is expressed at high levels in the dorsal spinal cord, in a pattern corresponding to the cell bodies of commissural neurons. Rat *robo1* is also expressed at lower levels in a subpopulation of ventral cells in the region of the developing motor column. Interestingly, this expression pattern is similar to and overlaps partly with the mRNA encoding DCC, another Ig superfamily member which is also expressed on commissural and motor neurons and encodes a receptor for Netrin-1 (Keino-Masu et al, 1996). Rat *robo1* is not, however, expressed in the either the floor plate or the roof plate of the spinal cord or in the dorsal root ganglia. This is in contrast to rat *cdo,* which is strongly expressed in the roof plate (KB, MT-L, and R. Krauss. In the periphery, rat *robo1* is also found to be expressed in the the myotome and developing limb, in a pattern reminiscent of *c-met* (Ebens et al, 1996), indicating that rat *robo1* may also be expressed by migrating muscle precursor cells. Therefore, like its Drosophila homologue, rat *roboI* RNA is expressed by both crossing and non-crossing populations of axons, indicating that it encodes the functional equivalent of D-Robo1.

Genetic Stocks. All eight independent *robo* alleles were isolated on chromosomes deficient for *Fasciclin III* as described in Seeger et al., 1993. Subsequent use of a duplication that includes *FasIII*, and recombination of the *robo* chromosomes, indicates that the *robo* phenotype is independent of the absence of *FasIII*. Deficiencies were obtained from the Drosophila stock center at Bloomington, Indiana.

Cloning and Molecular Analysis of the *robo* Genes. Start points for a molecular walk to *robo* were obtained from the Berkeley and Crete Drosophila Genome Projects. Chromosomal walking was performed using standard techniques to isolate cosmids from the Tamkun library (Tamkun et al., 1992). cDNAs were isolated from the Zinn 9-12 hour Drosophila embryo gt11 library (Zinn et al., 1988), and from a human fetal brain library (Stratagene). Northern blot of poly-A⁺RNA and reverse Northern blots were hybridized using sensitive Church conditions.

Sequencing of the cDNAs and genomic subclones was performed by the dideoxynucleotide chain termination method using Sequenase (USB) following the manufacturer's protocol and with the AutoRead kit or AutoCycle kit (Pharmacia) or by ³³P cycle sequencing. Reactions were analyzed on a Pharmacies LKB or ABI automated laser fluorescent DNA sequencers respectively. The cDNAs were sequenced completely on both strands. Sequence contigs were compiled using Lasergene, Intelligenetics, and AssemblyLIGN software (Kodak Eastman). Database searches were performed using BLAST (Altschuel et al., 1990).

A full length *D-robo1* cDNA was generated by ligating two partial cDNAs at an internal HpaI site and subcloning into the EcoRI site of pBluescript.SK+. A full length H-*robo1* cDNA was synthesized by ligating an XbaI-SalI fragment from a cDNA and a PCR product coding for the carboxy-terminal 222 amino acids at a Sail site. The PCR product has an EcoRI site introduced at the stop codon. The ligation product was cloned into pBluescript.SK+ digested with XbaI and EcoRI.

To clone the rat *robo1* cDNA, degenerate oligonucleotide primers designed against sequences conserved between the 5' ends of D-Robol and H-Robol were used to amplify a 500 bp fragment from an E13 rat brain cDNA by PCR. This fragment was used to screen an E13 spinal cord library at high stringency, resulting in the isolation of a 4.2 kb cDNA clone comprising all but the last 700 nucleotides. Subsequent screenings of the library with nonoverlapping probes from this cDNA led to the isolation of 4 partial and 7 full length clones. To clone the rat *robo2* cDNA, we screened the same library with a fragment of the *H-robo2* cDNA.

Expressed Sequence Tag and Genomic Sequences. The ESTs yu23d11 (#H77734), zr54g12 (#AA236414) and yq76e12 (#H52936, #H52937) code for portions of H-RoboI. The EST yq7e12 is aberrantly spliced to part of the human glycophorinB gene. Five ESTs yn50a07, yg02b06, yg17b06, yn13a04 and ym17g11 code for part of *H-robo2*. The Drosophila P1 clone DS00329 encodes the genomic sequence of *D-robo2*. Sequences 1825710 and 1825711 (both: #U88183; locus ZK377) code for the predicted sequence of C. elegans *robo.* The EST vi62e02 (#AA499193) codes for mouse *robo1*.

Identification of Molecular Defects In *robo* Alleles. Southern blots of *robo* alleles and their parental chromosomes were hybridized with fragments from the genomic cosmid clone 106-1435 or partial cDNA clones to identify restriction fragment length polymorphisms affecting the *robo* transcription unit. DNA was obtained from homozygous mutant embryos. 35 cycles of the PCR was subsequently performed on the DNA obtained from half an embryo. Primers specific for the region flanking the CfoI polymorphism used were : ROBO6 (5'-GCATTGGGTCATCTGTAGAG -3') and ROBO23 (5'-AGCTATCTGGAGGGAGGCAT-3'). The PCR products were purified on a Pharmacia H300 spin column and sequenced directly.

Transformation of Drosophila, *robo* Rescue, and Overexpression. The 16 kb XbaI fragment from cosmid 106-1435 was cloned into the Drosophila transformation vector pCaSpeR3. Transfbrmant lines were generated and mapped by standard procedures. Four independent lines were shown to rescue *robo*^{*1,3,5*} alleles as judged by MAb 1D4 staining.

PCR amplification of the D-robo ORF using the primers (5'-GAGTGGTGAATTCAACAGCACCAAAACCACAAAATGCATCCC-3') and (5'-CGGGGAGTCTAGAACACTTCATCCTTAGGTG-3') produced a PCR product with an altered ribosome binding site that more closely matches the Drosophila consensus (Cavener, 1987), and has only 21 bp of 5' UTR and no 3' UTR sequences. The PCR product was digested with EcoRI and XbaI and cloned into pBluescript (Stratagene) and subsequently, pUAST (Brand and Perrimon 1993). Transformant lines were crossed to *elav-GAL4* and *sca-GAL4* lines which express GAL4 in all neurons, or *ftzng-GAL4* which expresses in a subset of CNS neurons (Lin et al, 1994). Embryos were assayed by staining with MAbs BP102, 1D4 and 13C9. For ectopic expression in the *robo* mutant background, the stocks *robo*^{*3*} and *robo*^{*5*} (both protein nulls) were used. Crosses utilized the stocks *w; robo*/*CyO; UAS-robo* and *w; robo*/*CyO; elav-GAL4*. Due to the difficulty of maintaining a balanced stock, *robo*/*+;ftzngGAL4*/*+* males were generated as required.

Generation of Fusion Proteins and Antibodies. A six histidine tagged fusion protein was constructed by cloning amino acids 404-725 of the D-robo protein into the PstI site of the pQE31 vector (Qiagen). Fusion proteins were purified under denaturing conditions and subsequently dialyzed against PBS. Immunization of mice and MAb production followed standard protocols (Patel, 1994).

RNA Localization and Protein Immunocytochemistry. Digoxigenin labeled antisense *robo* transcripts were generated from a subclone of a *robo* cDNA in Bluescript. In-situ tissue hybridization was performed as described in Tear et al., 1996. Immunocytochemistry was performed as described by Patel, 1994. MAb ID4 was used at a dilution of 1:5 and BP102 at 1:10. For anti-robo staining, MAb 13C9 was diluted 1:10 in PBS with 0.1% Tween-20, and the embryos were fixed and cracked so as to minimize exposure to methanol. The presence of triton and storage of embryos in methanol were both found to destroy the activity of MAb 13C9.

In situ hybridization of rat spinal cords was carried out essentially as described in Fan and Tessier-Lavigne, 1994. E13 embryos were fixed in 4% paraformaldehyde, processed, embedded in OCT, and sectioned to 10 m. A 1.0kb ³⁵S antisense rRobo riboprobe spannning the the first three immunoglobulin domains was used for hybridization. An additional nonoverlapping probe was also used with identical results. DCC transcripts were detected as described in Keino-Masu et al., 1996. Immunohistochemistry against TAG-1 was carried out on 10 m transverse spinal cord sections using 4D7 monoclonal antibody (Dodd et al, 1988).

Electron Microscopy. Canton S embryos were hand devitellinized, opened dorsally to remove the gut, and prepared for immunoelectron microscopy according to the procedures described previously (Lin et al., 1994), with the following modifications. The fixed embryos were incubated sequentially with MAb 13C9 (1:1) for 1-2 hours, biotinylated goat anti-mouse secondary antibody (1:250) for 1.5 hours, and then streptavidin-conjugated HRP (1:200) for 1.5 hours. Hydrogen peroxide (0.01%) was used instead of glucose oxidase for the HRP-DAB reaction.

### References

Altman, J. and Bayer, S.A. (1984) Adv. Anat. Embryol. Cell Biol. 85, 1-164.

Altschul, S.F., et al. (1990) J. Mol. Biol. *215*, 403-410.

Bastiani, M.J., et al. (1987) Cell 48, 745-755.

Brand, A. H., and Perrimon, N. (1993) Development 118, 401-415.

Cavener, D. (1987) Nucl. Acids Res. 15, 1353-1361.

Chan, S. et al. (1996) Cell 87, 187-195.

Dodd, J., et al. (1988) Neuron 1, 105-116.

Ebens, A., et al. (1996) Neuron 17, 1157-1172.

Elkins, T., et al. (1990) Cell 60, 565-575.

Fan, C.M. and Tessier-Lavigne, M. (1994) Cell 79, 1175-1186.

Gertler, F.B., et al. (1995) Genes Develop. 9, 521-533.

Harris, R., Sabatelli, L.M., and Seeger, M.A. (1996) Neuron 17, 217-228.

Hedgecock, E.M., Culotti, J.G., and Hall, D.H. (1990) Neuron 4, 61-85.

Kang, J-S., et al. (1997) J. Cell Biol. 138, 203-213.

Keino-Masu, K., et al. (1996) Cell 87, 175-185.

Kennedy, T.E., et al. (1994) Cell 78, 425-435.

Kerrebrock, A. W., et al. (1995) Cell 83, 247-256.

Kidd, T., Russell, C., Goodman, C.S., and Tear, G. (1997). Dosage sensitive and

complementary functions of Roundabout and Commissureless control axon crossing of the CNS midline. Neuron, in review.

Kolodziej, P.A., et al. (1996) Cell 87, 197-204.

Kyte, J., and Doolittle, R.F. (1982) J. Mol. Biol. 157, 105-132.

Lin, D.M., et al. (1994) Neuron 13, 1055-1069.

Mitchell, K.J., et al. (1996) Neuron 17, 203-215.

Myers, P.Z., and Bastiani, M.J. (1993) Journal of Neuroscience 13, 127-143.

Niebuhr, K., et al. (1997) EMBO J. 16, 5433-5444.

Nielsen, H., et al. (1997) Protein Engineering 10, 1-6.

Patel, N. H. (1994) In "Methods in Cell Biology, Vol 44. Drosophila melanogaster: Practical Uses in Cell Biology" (L. S. B. Goldstein and E. Fyrberg, eds) Academic Press, New York. Seeger, M., Tear, G., Ferres-Marco, D., and Goodman C.S. (1993) Neuron 10, 409-426. Serafini, T., et al. (1994) Cell 78, 409-424.

Stoeckli, E.T., and Landmesser, L.T. (1995) Neuron 14, 1165-1179.

Stoeckli, et al. (1997) Neuron 18, 209-221.

Tamkun, J.W., et al. (1992) Cell 68, 561-572.

Tear, G., et al. (1993) Perspectives on Developmental Neurobiology 1, 183-194.

Tear G., et al. (1996) Neuron 16, 501-514.

Tessier-Lavigne, M., and Goodman, C.S. (1996) Science 274, 1123-1133.

Wadsworth, W.G., Bhatt, H., and Hedgecock, E.M. (1996) Neuron 16, 35-46.

Zallen, J., Yi, A., and Bargmann, C. (1997). The conserved immunoglobulin superfamily member SAX-3/Robo directs multiple aspects of axon guidance in C. elegans. Cell, in review.

Zinn, K., McAllister, L., and Goodman, C. S. (1988)Cell 53, 577-587.

Although the foregoing invention has been described in some detail by way of illustration and example for purposes of clarity of understanding, it will be readily apparent to those of ordinary skill in the art in light of the teachings of this invention that certain changes and modifications may be made thereto.

## Claims

1. An isolated polypeptide comprising SEQ ID NO: 2, 4, 6, 8 or 10.

2. An isolated polypeptide comprising SEQ ID NO: 8, or a fragment thereof comprising residues 1-12, residues 18-28, residues 31-40 or residues 45-65 of SEQ ID NO: 8.

3. An isolated polypeptide comprising SEQ ID NO: 10, or a fragment thereof comprising residues 5-16, residues 38-47, residues 83-94, residues 112-125, residues 168-180, residues 195-209, residues 222-235 or residues 241-254 of SEQ ID NO: 10.

4. An isolated recombinant nucleic acid joined to nucleotide(s) other than that which it is joined to on a natural chromosome, comprising a coding strand encoding a polypeptide according to claim 1, 2 or 3.

5. A cell comprising a nucleic acid according to claim 4.

6. A method of making a Robo polypeptide, comprising the steps of:
incubating a host cell or cellular extract containing a nucleic acid according to claim 4 under conditions whereby the polypeptide encoded by the nucleic acid is expressed and recovering the expressed polypeptide.

7. A method for modulating function or morphology of a target cell in vitro, the method comprising providing the target cell in culture with a polypeptide comprising SEQ ID No: 2, 4, 6, 8, 10, or an antibody specific for said polypeptide.

8. A recombinant nucleic acid comprising a strand of SEQ ID NO: 1, 3, 5, 7 or 9, wherein said strand is flanked by fewer than 500 bp of native flanking sequence.

9. An antibody specific for a polypeptide according to claim 1, 2 or 3.

## Patentansprüche

1. Ein isoliertes Polypeptid, welches die SEQ ID NR: 2, 4, 6, 8 oder 10 umfasst.

2. Ein isoliertes Polypeptid, welches die SEQ ID NR: 8 umfasst, oder ein Fragment von diesem, welches die Reste 1-12, die Reste 18-28, die Reste 31-40 oder die Reste 45-65 der SEQ ID NR: 8 umfasst.

3. Ein isoliertes Polypeptid, welches die SEQ ID NR: 10 umfasst, oder ein Fragment von diesem, welches die Reste 5-16, die Reste 38-47, die Reste 83-94, die Reste 112-125, die Reste 168-180, die Reste 195-209, die Reste 222-235 oder die Reste 241-254 der SEQ ID NR: 10 umfasst.

4. Eine isolierte rekombinante Nukleinsäure, die mit einem Nukleotid (Nukleotiden) verknüpft ist, das (die) von dem verschieden ist (sind), mit dem sie auf einem natürlichen Chromosom verknüpft ist, welche einen codierenden Strang umfasst, der ein Polypeptid gemäß Anspruch 1, 2 oder 3 codiert.

5. Eine Zelle, welche eine Nukleinsäure gemäß Anspruch 4 umfasst.

6. Ein Verfahren zur Erzeugung eines Robo-Polypeptids, welches die folgenden Schritte umfasst:
Inkubieren einer Wirtszelle oder eines Zellextrakts, welcher eine Nukleinsäure gemäß Anspruch 4 enthält, unter Bedingungen, durch welche das Polypeptid, das durch die Nukleinsäure codiert wird, exprimiert wird, und Gewinnen des exprimierten Polypeptids.

7. Ein Verfahren zum Modulieren der Funktion oder Morphologie einer Zielzelle in vitro, wobei das Verfahren das Bereitstellen der Zielzelle in Kultur mit einem Polypeptid, das die SEQ ID NR: 2, 4, 6, 8, 10 umfasst, oder einem Antikörper, der für das Polypeptid spezifisch ist, umfasst.

8. Eine rekombinante Nukleinsäure, welche einen Strang der SEQ ID NR: 1, 3, 5, 7 oder 9 umfasst, wobei der Strang durch weniger als 500 bp der nativen flankierenden Sequenz flankiert ist.

9. Ein Antikörper, der für ein Polypeptid gemäß Anspruch 1, 2 oder 3 spezifisch ist.

## Revendications

1. Polypeptide isolé comprenant la séquence SEQ ID No. 2, 4, 6, 8 ou 10.

2. Polypeptide isolé comprenant la séquence SEQ ID No. 8, ou fragment d'un tel polypeptide comprenant les restes 1-12, les restes 18-28, les restes 31-40 ou les restes 45-65 de la séquence SEQ ID No. 8.

3. Polypeptide isolé comprenant la séquence SEQ ID No. 10, ou un fragment d'un tel polypeptide comprenant les restes 5-16, les restes 38-47, les restes 83-94, les restes 112-125, les restes 168-180, les restes 195-209, les restes 222-235 ou les restes 241-254 de la séquence SEQ ID No. 10.

4. Acide nucléique recombinant isolé, lié à un nucléotide ou des nucléotides autres que ceux auxquels il est lié sur un chromosome naturel, comprenant un brin codant qui code pour un polypeptide selon l'une quelconque des revendications 1 à 3.

5. Cellule comprenant un acide nucléique selon la revendication 4.

6. Procédé de préparation d'un polypeptide Robo, qui comprend les étapes consistant à incuber une cellule-hôte ou un extrait cellulaire contenant un acide nucléique selon la revendication 4, dans des conditions dans lesquelles le polypeptide codé par l'acide nucléique est exprimé, et récupérer le polypeptide exprimé.

7. Procédé pour moduler la fonction ou la morphologie d'une cellule-cible *in vitro,* qui comprend la fourniture de la cellule-cible dans une culture avec un polypeptide comprenant la séquence SEQ ID No. 2, 4, 6, 8 ou 10, ou un anticorps spécifique dudit polypeptide.

8. Acide nucléique recombinant, comprenant un brin de séquence SEQ ID No. 1, 3, 5, 7 ou 9, ledit brin étant entouré de moins de 500 bp de séquence de flanquement naturelle.

9. Anticorps spécifique d'un polypeptide selon l'une quelconque des revendications 1 à 3.


### SEQUENCE LISTING

(1) GENERAL INFORMATION:
   (i) APPLICANT: Goodman, Corey S.
      Kidd, Thomas
      Mitchell, Kevin
      Tear, Guy
   (ii) TITLE OF INVENTION: Robo: A Novel Family of Polypeptide and
      Nucleic Acids
   (iii) NUMBER OF SEQUENCES: 12
   (iv) CORRESPONDENCE ADDRESS:
      (A) ADDRESSEE: SCIENCE & TECHNOLOGY LAW GROUP
      (B) STREET: 75 DENISE DRIVE
      (C) CITY: HILLSBOROUGH
      (D) STATE: CALIFORNIA
      (E) COUNTRY: USA
      (F) ZIP: 94010
   (V) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: OSMAN, RICHARD A
      (B) REGISTRATION NUMBER: 36,627
      (C) REFERENCE/DOCKET NUMBER: B98-006
   (ix) TELECOMMUNICATION INFORMATION:
      (A) TELEPHONE: (650) 343-4341
      (B) TELEFAX: (650) 343-4342
(2) INFORMATION FOR SEQ ID NO:1:
   (i) SEQUENCE CHARACTERISTICS:
      (A) LENGTH: 4188 base pairs
      (B) TYPE: nucleic acid
      (C) STRANDEDNESS: double
      (D) TOPOLOGY: linear
   (ii) MOLECULE TYPE: cDNA

   (xi) SEQUENCE DESCRIPTION: SEQ ID NO:1:
   ATGCATCCCA TGCATCCCGA AAACCACGCC ATCGCCCGGA GCACGAGCAC CACTAATAAC 60
   CCATCTCGCA GTCGGAGCAG CAGGATGTGG CTCCTGCCCG CCTGGCTGCT CCTCGTCCTG 120
   GTGGCCAGCA ATGGCCTGCC AGCAGTCAGA GGCCAGTACC AATCGCCACG TATCATCGAG 180
   CATCCCACGG ATCTGGTCGT TAAGAAGAAT GAACCCGCCA CGCTCAACTG CAAAGTGGAG 240
   GGCAAGCCGG AACCCACCAT TGAGTGGTTT AAGGATGGCG AACCCGTCAG CACCAACGAA 300
   AAGAAATCGC ACCGCGTCCA GTTCAAGGAC GGCGCCCTCT TCTTTTACAG GACAATGCAA 360
   GGCAAGAAGG AGCAGGACGG CGGAGAGTAC TGGTGCGTGG CCAAGAACCG AGTGGGCCAG 420
   GCCGTTAGTC GCCATGCCTC CCTCCAGATA GCTGTTTTGC GCGACGATTT TCGCGTGGAG 480
   CCCAAAGACA CGCGAGTGGC CAAAGGCGAG ACGGCTCTGC TGGAGTGTGG GCCGCCCAAA 540
   GGCATTCCAG AGCCAACGCT GATTTGGATA AAGGACGGCG TTCCCTTGGA CGACCTGAAA 600
   GCCATGTCGT TTGGCGCCAG CTCCCGCGTT CGAATTGTGG ACGGTGGCAA CCTGCTGATC 660
   AGCAATGTGG AGCCCATTGA TGAGGGCAAC TACAAGTGCA TTGCCCAGAA TCTGGTAGGC 720
   ACCCGCGAGA GCAGCTATGC CAAGCTGATT GTCCAGGTCA AACCATACTT TATGAAGGAG 780
   CCCAAGGATC AGGTGATGCT CTACGGCCAG ACAGCCACTT TCCACTGCTC AGTGGGCGGT 840
   GATCCGCCGC CGAAAGTGTT GTGGAAAAAG GAGGAGGGCA ATATTCCGGT GTCCAGAGCG 900
   CGAATCCTTC ACGACGAGAA AAGTTTAGAG ATATCCAACA TAACGCCCAC CGATGAGGGC 960
   ACCTATGTCT GCGAGGCACA CAACAATGTC GGTCAGATCA GCGCTAGGGC TTCTCTTATA 1020
   GTCCACGCTC CGCCGAACTT TACGAAAAGA CCCAGTAACA AGAAAGTGGG ACTAAATGGG 1080
   GTTGTCCAAC TACCTTGCAT GGCCTCCGGA AACCCTCCGC CGTCTGTATT CTGGACCAAG 1140
   GAAGGAGTAT CCACTCTTAT GTTCCCAAAT AGTTCGCACG GAAGGCAGTA TGTGGCTGCC 1200
   GATGGAACTC TGCAGATTAC GGATGTGCGG CAGGAAGACG AAGGCTACTA TGTGTGTTCC 1260
   GCTTTCAGTG TAGTCGATTC CTCTACAGTA CGGGTTTTCC TGCAAGTCAG CTCGGTAGAC 1320
   GAGCGTCCAC CTCCGATTAT TCAAATCGGA CCTGCCAATC AAACACTGCC CAAGGGATCA 1380
   GTTGCTACTT TACCCTGTCG GGCCACTGGA AATCCCAGTC CCCGTATCAA GTGGTTCCAC 1440
   GATGGACATG CCGTACAAGC GGGCAATCGA TACAGCATCA TCCAAGGAAG CTCACTGAGA 1500
   GTCGATGACC TTCAACTAAG TGACTCTGGT ACCTACACCT GCACTGCATC TGGCGAACGA 1560
   GGAGAAACTT CCTGGGCTGC CACACTAACG GTGGAAAAAC CCGGTTCTAC ATCTCTTCAC 1620
   CGGGCAGCTG ATCCTAGCAC TTATCCTGCT CCTCCAGGAA CACCTAAAGT CCTGAATGTC 1680
   AGTCGCACCA GCATTAGTCT TCGTTGGGCT AAAAGCCAAG AGAAACCCGG AGCTGTGGGC 1740
   CCAATCATTG GATACACTGT AGAGTACTTC AGTCCGGATC TGCAAACTGG TTGGATTGTG 1800
   GCTGCCCATC GAGTCGGCGA CACTCAAGTC ACTATCTCGG GTCTCACTCC TGGCACTTCG 1860
   TATGTGTTCC TAGTTAGAGC TGAGAATACT CAGGGTATTT CTGTGCCTTC CGGCTTATCA 1920
   AATGTTATTA AAACCATTGA GGCAGATTTC GATGCAGCTT CTGCCAATGA TTTGTCAGCA 1980
   GCTCGAACTT TGCTGACAGG AAAGTCGGTG GAGCTAATAG ATGCCTCGGC TATCAATGCT 2040
   AGTGCCGTTA GACTTGAGTG GATGCTCCAC GTGAGCGCTG ATGAGAAATA CGTAGAGGGC 2100
   CTGCGCATAC ACTATAAGGA TGCCAGTGTA CCATCCGCAC AGTATCACTC GATCACTGTT 2160
   ATGGATGCCT CTGCAGAATC GTTTGTGGTG GGAAACCTTA AGAAGTACAC CAAGTATGAG 2220
   TTCTTCCTAA CACCCTTTTT TGAGACAATT GAAGGACAGC CCAGTAACTC CAAGACAGCC 2280
   CTCACCTATG AAGATGTTCC CTCCGCACCA CCGGATAACA TTCAGATTGG CATGTACAAC 2340
   CAAACAGCCG GTTGGGTGCG TTGGACTCCG CCACCCTCCC AGCACCACAA TGGCAATTTG 2400
   TATGGCTACA AGATTGAGGT CAGCGCCGGT AACACCATGA AGGTGCTGGC CAATATGACT 2460
   CTTAATGCTA CCACCACATC TGTGCTCCTA AATAACCTAA CCACCGGAGC TGTGTACAGC 2520
   GTGAGGTTGA ACTCCTTTAC CAAGGCAGGA GATGGACCTT ACTCCAAACC GATATCACTA 2580
   TTCATGGACC CCACCCATCA TGTGCATCCG CCACGGGCAC ATCCAAGCGG CACCCATGAT 2640
   GGGCGACATG AGGGACAGGA TCTCACGTAT CATAACAATG GCAACATACC ACCTGGCGAC 2700
   ATTAATCCCA CCACTCATAA AAAGACCACT GACTACCTAT CTGGACCGTG GCTAATGGTG 2760
   CTGGTCTGCA TCGTTCTTCT AGTCCTGGTT ATTTCGGCGG CTATTTCGAT GGTCTACTTC 2820
   AAGCGCAAGC ATCAAATGAC CAAGGAATTG GGTCACTTAA GTGTGGTCAG TGACAACGAA 2880
   ATAACCGCAT TAAATATCAA TAGCAAAGAG AGCCTTTGGA TAGACCATCA TCGTGGATGG 2940
   CGAACTGCCG ATACTGACAA AGACTCAGGA TTAAGCGAAT CGAAGCTACT ATCCCACGTT 3000
   AACAGCAGTC AATCCAACTA CAATAACTCC GATGGAGGAA CCGATTATGC AGAAGTTGAC 3060
   ACCCGTAACC TTACCACCTT CTACAATTGT CGCAAGAGCC CCGATAATCC CACGCCGTAC 3120
   GCCACCACTA TGATCATTGG TACCTCTTCC AGTGAGACCT GCACCAAGAC AACATCTATA 3180
   AGTGCCGATA AGGACTCGGG AACTCATTCG CCCTATTCTG ACGCATTTGC CGGTCAGGTG 3240
   CCAGCGGTTC CTGTTGTCAA ATCCAACTAT CTTCAGTATC CGGTTGAACC GATCAACTGG 3300
   TCAGAGTTTC TACCCCCGCC GCCAGAACAC CCACCTCCGT CTTCTACCTA TGGATACGCA 3360
   CAAGGATCTC CTGAATCTTC GCGGAAGAGC TCCAAAAGCG CAGGTTCCGG CATTTCTACA 3420
   AATCAAAGCA TTCTGAACGC ATCCATACAC AGCAGCTCCT CGGGCGGCTT TTCAGCTTGG 3480
   GGAGTATCGC CCCAATATGC TGTCGCCTGT CCACCGGAAA ACGTTTATAG CAATCCGCTG 3540
   TCGGCAGTGG CTGGCGGCAC CCAGAACCGC TATCAGATAA CGCCCACAAA CCAACATCCG 3600
   CCACAGTTAC CGGCCTACTT TGCCACCACG GGTCCAGGAG GAGCTGTACC ACCCAACCAC 3660
   CTGCCATTTG CCACACAGCG TCATGCAGCC AGCGAGTACC AGGCTGGACT GAATGCAGCG 3720
   CGATGTGCCC AAAGCCGCGC CTGCAACAGC TGCGATGCCT TGGCCACACC CTCGCCCATG 3780
   CAACCCCCAC CGCCAGTTCC CGTACCCGAG GGCTGGTACC AACCGGTGCA TCCCAATAGC 3840
   CACCCGATGC ACCCGACCTC CTCCAACCAC CAGATCTACC AGTGCTCCTC CGAGTGCTCG 3900
   GATCACTCGA GGAGCTCGCA GAGTCACAAG CGGCAGCTGC AGCTCGAGGA GCACGGCAGC 3960
   AGTGCCAAAC AACGCGGAGG ACACCACCGT CGACGAGCCC CGGTGGTGCA GCCGTGCATG 4020
   GAGAGCGAGA ACGAGAACAT GCTGGCGGAG TACGAGCAGC GCCAGTACAC CAGCGATTGC 4080
   TGCAATAGCT CCCGCGAGGG CGACACCTGC TCCTGCAGCG AGGGATCCTG TCTTTACGCC 4140
   GAGGCGGGCG AGCCGGCGCC TCGTCAAATG ACTGCTAAGA ACACCTAA 4188
(2) INFORMATION FOR SEQ ID NO:2:
   (i) SEQUENCE CHARACTERISTICS:
      (A) LENGTH: 1395 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Met His Pro Met His Pro Glu Asn His Ala Ile Ala Arg Ser Thr Ser
   1               5                   10                  15
   Thr Thr Asn Asn Pro Ser Arg Ser Arg Ser Ser Arg Met Trp Leu Leu
               20                  25                  30
   Pro Ala Trp Leu Leu Leu Val Leu Val Ala Ser Asn Gly Leu Pro Ala
           35                  40                  45
   Val Arg Gly Gln Tyr Gln Ser Pro Arg Ile Ile Glu His Pro Thr Asp
       50                  55                  60
   Leu Val Val Lys Lys Asn Glu Pro Ala Thr Leu Asn Cys Lys Val Glu
   65                  70                  75                  80
   Gly Lys Pro Glu Pro Thr Ile Glu Trp Phe Lys Asp Gly Glu Pro Val
                   85                  90                  95
   Ser Thr Asn Glu Lys Lys Ser His Arg Val Gln Phe Lys Asp Gly Ala
               100                 105                 110
   Leu Phe Phe Tyr Arg Thr Met Gln Gly Lys Lys Glu Gln Asp Gly Gly
           115                 120                 125
   Glu Tyr Trp Cys Val Ala Lys Asn Arg Val Gly Gln Ala Val Ser Arg
       130                 135                 140
   His Ala Ser Leu Gln Ile Ala Val Leu Arg Asp Asp Phe Arg Val Glu
   145                 150                 155                 160
   Pro Lys Asp Thr Arg Val Ala Lys Gly Glu Thr Ala Leu Leu Glu Cys
                   165                 170                 175
   Gly Pro Pro Lys Gly Ile Pro Glu Pro Thr Leu Ile Trp Ile Lys Asp
               180                 185                 190
   Gly Val Pro Leu Asp Asp Leu Lys Ala Met Ser Phe Gly Ala Ser Ser
           195                 200                 205
   Arg Val Arg Ile Val Asp Gly Gly Asn Leu Leu Ile Ser Asn Val Glu
       210                 215                 220
   Pro Ile Asp Glu Gly Asn Tyr Lys Cys Ile Ala Gln Asn Leu Val Gly
   225                 230                 235                 240
   Thr Arg Glu Ser Ser Tyr Ala Lys Leu Ile Val Gln Val Lys Pro Tyr
                   245                 250                 255
   Phe Met Lys Glu Pro Lys Asp Gln Val Met Leu Tyr Gly Gln Thr Ala
   260 265 270
   Thr Phe His Cys Ser Val Gly Gly Asp Pro Pro Pro Lys Val Leu Trp
   275 280 285
   Lys Lys Glu Glu Gly Asn Ile Pro Val Ser Arg Ala Arg Ile Leu His
   290 295 300
   Asp Glu Lys Ser Leu Glu Ile Ser Asn Ile Thr Pro Thr Asp Glu Gly
   305 310 315 320
   Thr Tyr Val Cys Glu Ala His Asn Asn Val Gly Gln Ile Ser Ala Arg
   325 330 335
   Ala Ser Leu Ile Val His Ala Pro Pro Asn Phe Thr Lys Arg Pro Ser
   340 345 350
   Asn Lys Lys Val Gly Leu Asn Gly Val Val Gln Leu Pro Cys Met Ala
   355 360 365
   Ser Gly Asn Pro Pro Pro Ser Val Phe Trp Thr Lys Glu Gly Val Ser
   370 375 380
   Thr Leu Met Phe Pro Asn Ser Ser His Gly Arg Gln Tyr Val Ala Ala
   385 390 395 400
   Asp Gly Thr Leu Gln Ile Thr Asp Val Arg Gln Glu Asp Glu Gly Tyr
   405 410 415
   Tyr Val Cys Ser Ala Phe Ser Val Val Asp Ser Ser Thr Val Arg Val
   420 425 430
   Phe Leu Gln Val Ser Ser Val Asp Glu Arg Pro Pro Pro Ile Ile Gln
   435 440 445
   Ile Gly Pro Ala Asn Gln Thr Leu Pro Lys Gly Ser Val Ala Thr Leu
   450 455 460
   Pro Cys Arg Ala Thr Gly Asn Pro Ser Pro Arg Ile Lys Trp Phe His
   465 470 475 480
   Asp Gly His Ala Val Gln Ala Gly Asn Arg Tyr Ser Ile Ile Gln Gly
   485 490 495
   Ser Ser Leu Arg Val Asp Asp Leu Gln Leu Ser Asp Ser Gly Thr Tyr
   500 505 510
   Thr Cys Thr Ala Ser Gly Glu Arg Gly Glu Thr Ser Trp Ala Ala Thr
   515 520 525
   Leu Thr Val Glu Lys Pro Gly Ser Thr Ser Leu His Arg Ala Ala Asp
   530 535 540
   Pro Ser Thr Tyr Pro Ala Pro Pro Gly Thr Pro Lys Val Leu Asn Val
   545 550 555 560
   Ser Arg Thr Ser Ile Ser Leu Arg Trp Ala Lys Ser Gln Glu Lys Pro
   565 570 575
   Gly Ala Val Gly Pro Ile Ile Gly Tyr Thr Val Glu Tyr Phe Ser Pro
   580 585 590
   Asp Leu Gln Thr Gly Trp Ile Val Ala Ala His Arg Val Gly Asp Thr
   595 600 605
   Gln Val Thr Ile Ser Gly Leu Thr Pro Gly Thr Ser Tyr Val Phe Leu
   610 615 620
   Val Arg Ala Glu Asn Thr Gln Gly Ile Ser Val Pro Ser Gly Leu Ser
   625 630 635 640
   Asn Val Ile Lys Thr Ile Glu Ala Asp Phe Asp Ala Ala Ser Ala Asn
   645 650 655
   Asp Leu Ser Ala Ala Arg Thr Leu Leu Thr Gly Lys Ser Val Glu Leu
   660 665 670
   Ile Asp Ala Ser Ala Ile Asn Ala Ser Ala Val Arg Leu Glu Trp Met
   675 680 685
   Leu His Val Ser Ala Asp Glu Lys Tyr Val Glu Gly Leu Arg Ile His
   690 695 700
   Tyr Lys Asp Ala Ser Val Pro Ser Ala Gln Tyr His Ser Ile Thr Val
   705 710 715 720
   Met Asp Ala Ser Ala Glu Ser Phe Val Val Gly Asn Leu Lys Lys Tyr
   725 730 735
   Thr Lys Tyr Glu Phe Phe Leu Thr Pro Phe Phe Glu Thr Ile Glu Gly
   740 745 750
   Gln Pro Ser Asn Ser Lys Thr Ala Leu Thr Tyr Glu Asp Val Pro Ser
   755 760 765
   Ala Pro Pro Asp Asn Ile Gln Ile Gly Met Tyr Asn Gln Thr Ala Gly
   770 775 780
   Trp Val Arg Trp Thr Pro Pro Pro Ser Gln His His Asn Gly Asn Leu
   785 790 795 800
   Tyr Gly Tyr Lys Ile Glu Val Ser Ala Gly Asn Thr Met Lys Val Leu
   805 810 815
   Ala Asn Met Thr Leu Asn Ala Thr Thr Thr Ser Val Leu Leu Asn Asn
   820 825 830
   Leu Thr Thr Gly Ala Val Tyr Ser Val Arg Leu Asn Ser Phe Thr Lys
   835 840 845
   Ala Gly Asp Gly Pro Tyr Ser Lys Pro Ile Ser Leu Phe Met Asp Pro
   850 855 860
   Thr His His Val His Pro Pro Arg Ala His Pro Ser Gly Thr His Asp
   865 870 875 880
   Gly Arg His Glu Gly Gln Asp Leu Thr Tyr His Asn Asn Gly Asn Ile
   885 890 895
   Pro Pro Gly Asp Ile Asn Pro Thr Thr His Lys Lys Thr Thr Asp Tyr
   900 905 910
   Leu Ser Gly Pro Trp Leu Met Val Leu Val Cys Ile Val Leu Leu Val
   915 920 925
   Leu Val Ile Ser Ala Ala Ile Ser Met Val Tyr Phe Lys Arg Lys His
   930 935 940
   Gln Met Thr Lys Glu Leu Gly His Leu Ser Val Val Ser Asp Asn Glu
   945 950 955 960
   Ile Thr Ala Leu Asn Ile Asn Ser Lys Glu Ser Leu Trp Ile Asp His
   965 970 975
   His Arg Gly Trp Arg Thr Ala Asp Thr Asp Lys Asp Ser Gly Leu Ser
   980 985 990
   Glu Ser Lys Leu Leu Ser His Val Asn Ser Ser Gln Ser Asn Tyr Asn
   995 1000 1005
   Asn Ser Asp Gly Gly Thr Asp Tyr Ala Glu Val Asp Thr Arg Asn Leu
   1010 1015 1020
   Thr Thr Phe Tyr Asn Cys Arg Lys Ser Pro Asp Asn Pro Thr Pro Tyr
   1025 1030 1035 1040
   Ala Thr Thr Met Ile Ile Gly Thr Ser Ser Ser Glu Thr Cys Thr Lys
   1045 1050 1055
   Thr Thr Ser Ile Ser Ala Asp Lys Asp Ser Gly Thr His Ser Pro Tyr
   1060 1065 1070
   Ser Asp Ala Phe Ala Gly Gln Val Pro Ala Val Pro Val Val Lys Ser
   1075 1080 1085
   Asn Tyr Leu Gln Tyr Pro Val Glu Pro Ile Asn Trp Ser Glu Phe Leu
   1090 1095 1100
   Pro Pro Pro Pro Glu His Pro Pro Pro Ser Ser Thr Tyr Gly Tyr Ala
   1105 1110 1115 1120
   Gln Gly Ser Pro Glu Ser Ser Arg Lys Ser Ser Lys Ser Ala Gly Ser
   1125 1130 1135
   Gly Ile Ser Thr Asn Gln Ser Ile Leu Asn Ala Ser Ile His Ser Ser
   1140 1145 1150
   Ser Ser Gly Gly Phe Ser Ala Trp Gly Val Ser Pro Gln Tyr Ala Val
   1155 1160 1165
   Ala Cys Pro Pro Glu Asn Val Tyr Ser Asn Pro Leu Ser Ala Val Ala
       1170                1175                1180
   Gly Gly Thr Gln Asn Arg Tyr Gln Ile Thr Pro Thr Asn Gln His Pro
   1185                1190                1195                1200
   Pro Gln Leu Pro Ala Tyr Phe Ala Thr Thr Gly Pro Gly Gly Ala Val
                   1205                1210                1215
   Pro Pro Asn His Leu Pro Phe Ala Thr Gln Arg His Ala Ala Ser Glu
               1220                1225                1230
   Tyr Gln Ala Gly Leu Asn Ala Ala Arg Cys Ala Gln Ser Arg Ala Cys
           1235                1240                1245
   Asn Ser Cys Asp Ala Leu Ala Thr Pro Ser Pro Met Gln Pro Pro Pro
       1250                1255                1260
   Pro Val Pro Val Pro Glu Gly Trp Tyr Gln Pro Val His Pro Asn Ser
   1265                1270                1275                1280
   His Pro Met His Pro Thr Ser Ser Asn His Gln Ile Tyr Gln Cys Ser
                   1285                1290                1295
   Ser Glu Cys Ser Asp His Ser Arg Ser Ser Gln Ser His Lys Arg Gln
               1300                1305                1310
   Leu Gln Leu Glu Glu His Gly Ser Ser Ala Lys Gln Arg Gly Gly His
           1315                1320                1325
   His Arg Arg Arg Ala Pro Val Val Gln Pro Cys Met Glu Ser Glu Asn
       1330                1335                1340
   Glu Asn Met Leu Ala Glu Tyr Glu Gln Arg Gln Tyr Thr Ser Asp Cys
   1345                1350                1355                1360
   Cys Asn Ser Ser Arg Glu Gly Asp Thr Cys Ser Cys Ser Glu Gly Ser
                   1365                1370                1375
   Cys Leu Tyr Ala Glu Ala Gly Glu Pro Ala Pro Arg Gln Met Thr Ala
               1380                1385                1390
   Lys Asn Thr
           1395
(2) INFORMATION FOR SEQ ID NO:3:
   (i) SEQUENCE CHARACTERISTICS:
      (A) LENGTH: 4146 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:3:
   GGTGAAAATC CACGCATCAT CGAGCATCCC ATGGACACGA CGGTGCCAAA AAATGATCCA 60
   TTTACGTTTA ATTGCCAGGC CGAGGGCAAT CCAACACCAA CCATTCAATG GTTTAAGGAC 120
   GGTCGCGAAC TGAAGACGGA TACGGGTTCG CATCGCATAA TGCTGCCCGC CGGGGGTCTA 180
   TTCTTTCTCA AGGTTATCCA CTCACGTAGA GAGAGCGATG CGGGCACTTA CTGGTGCGAG 240
   GCCAAAAACG AGTTTGGAGT GGCACGGTCC AGGAATGCAA CGTTGCAAGT GGCAGTTCTC 300
   CGCGACGAAT TCCGTTTGGA GCCGGCAAAT ACCCGCGTGG CCCAAGGCGA GGTGGCCCTG 360
   ATGGAATGCG GTGCCCCCCG AGGATCTCCG GAGCCGCAAA TCTCGTGGCG CAAGAACGGC 420
   CAGACCCTGA ATCTTGTCGG GAACAAGCGG ATTCGCATTG TCGACGGTGG CAATCTGGCC 480
   ATCCAGGAAG CCCGCCAATC GGACGACGGA CGCTACCAGT GTGTGGTCAA GAATGTGGTT 540
   GGCACCCGGG AGTCGGCCAC CGCTTTTCTT AAAGTGCATG TACGTCCATT CCTCATCCGA 600
   GGACCCCAGA ATCAGACGGC GGTGGTGGGC AGCTCGGTGG TCTTCCAGTG CCGCATCGGA 660
   GGCGATCCCC TGCCTGATGT CCTGTGGCGA CGCACTGCCT CCGGCGGCAA TATGCCACTG 720
   CGTAAGTTTT CTTGGCTTCA TTCAGCTTCA GGTCGTGTGC ACGTACTTGA GGACCGCAGT 780
   CTGAAGCTGG ACGACGTTAC TCTGGAGGAC ATGGGCGAGT ACACTTGCGA GGCGGACAAT 840
   GCGGTGGGCG GCATCACGGC CACTGGCATC CTCACCGTTC ACGCTCCCCC CAAATTTGTG 900
   ATACGCCCCA AGAATCAGCT GGTGGAGATC GGTGATGAAG TGCTGTTCGA GTGCCAAGCG 960
   AATGGACATC CCCGACCAAC GCTCTACTGG TCGGTGGAGG GCAACAGCTC CCTGCTGCTC 1020
   CCCGGCTATC GGGATGGCCG CATGGAAGTG ACCCTGACGC CCGAGGGGCG CTCGGTGCTC 1080
   TCGATAGCTC GATTTGCCCG TGAGGATTCC GGAAAGGTGG TCACTTGCAA CGCCCTGAAC 1140
   GCCGTGGGCA GCGTCAGCAG TCGGACTGTG GTCAGTGTGG ATACGCAATT CGAGCTGCCA 1200
   CCGCCGATTA TCGAACAGGG GCCCGTGAAT CAAACGTTGC CCGTTAAATC AATTGTGGTT 1260
   CTGCCATGCC GAACTCTGGG CACTCCAGTG CCACAGGTCT CTTGGTACCT GGATGGCATA 1320
   CCCATCGATG TGCAGGAGCA CGAGCGGCGG AATCTTTCGG ACGCTGGAGC CTTAACCATT 1380
   TCGGATCTTC AGCGCCACGA GGATGAAGGC TTGTACACCT GCGTGGCCAG CAATCGCAAC 1440
   GGAAAATCCT CTTGGAGTGG TTACCTTCGT CTGGACACCC CGACAAATCC GAATATCAAG 1500
   TTCTTCAGAG CCCCAGAACT TTCCACCTAC CCAGGGCCGC CAGGAAAACC GCAAATGGTG 1560
   GAGAAGGGCG AAAATTCGGT GACTCTCAGC TGGACGAGGA GCAACAAGGT GGGCGGCTCC 1620
   AGTCTGGTGG GCTATGTAAT CGAGATGTTT GGCAAAAACG AAACGGATGG CTGGGTGGCT 1680
   GTGGGCACTA GGGTGCAAAA TACCACGTTT ACCCAAACGG GTCTGCTGCC GGGTGTGAAT 1740
   TACTTCTTTC TAATTCGAGC CGAGAACTCC CATGGCTTAT CACTGCCCAG TCCGATGTCG 1800
   GAACCCATTA CGGTGGGAAC GCGCTACTTC AATAGTGGTC TGGATCTGAG CGAGGCTCGT 1860
   GCCAGTCTGC TGTCCGGAGA TGTTGTGGAG CTGAGCAACG CCAGTGTGGT GGACTCCACT 1920
   AGCATGAAAC TCACCTGGCA GATCATCAAT GGCAAATACG TCGAGGGCTT CTATGTCTAT 1980
   GCGAGACAGT TGCCAAATCC AATAGTCAAC AATCCGGCGC CCGTTACTAG CAATACCAAT 2040
   CCGCTGCTGG GCTCTACATC CACATCCGCA TCCGCATCCG CCTCGGCATC GGCATTGATT 2100
   TCGACAAAGC CAAATATTGC AGCTGCCGGC AAACGTGATG GGGAGACAAA CCAGAGTGGA 2160
   GGAGGAGCTC CGACCCCACT GAACACCAAG TATCGCATGC TAACGATTCT CAATGGCGGT 2220
   GGCGCCTCAT CCTGCACCAT CACCGGGCTC GTCCAGTACA CGCTGTATGA ATTTTTCATC   2280
   GTGCCATTTT ACAAATCCGT CGAGGGCAAG CCGTCGAATT CGCGCATCGC TCGCACCCTT   2340
   GAAGATGTTC CCTCTGAGGC ACCATATGGA ATGGAGGCTC TGCTGTTGAA CTCCTCCGCG   2400
   GTCTTCCTCA AATGGAAGGC ACCAGAACTC AAGGATCGGC ATGGTGTTCT CTTGAACTAT   2460
   CATGTTATAG TCCGAGGTAT TGACACTGCC CACAATTTCT CACGCATTTT GACAAATGTC   2520
   ACCATCGATG CCGCTTCGCC TACTCTGGTT TTGGCCAATC TCACCGAAGG CGTCATGTAC   2580
   ACCGTGGGCG TGGCGGCCGG AAATAACGCT GGAGTTGGTC CTTATTGTGT CCCAGCTACT   2640
   TTGCGTTTGG ATCCCATCAC AAAGCGACTC GATCCGTTCA TCAATCAGCG GGACCATGTT   2700
   AACGATGTGC TGACGCAGCC CTGGTTCATA ATACTCCTGG GCGCCATCCT GGCCGTTCTT   2760
   ATGCTGTCCT TTGGCGCAAT GGTCTTTGTG AAGCGCAAGC ACATGATGAT GAAGCAGTCG   2820
   GCCCTAAATA CAATGCGTGG CAATCACACG AGCGACGTGC TCAAAATGCC GAGTCTATCG   2880
   GCGCGCAATG GAAACGGCTA CTGGCTGGAC TCCTCCACCG GCGGAATGGT GTGGCGTCCC   2940
   TCGCCCGGCG GCGACTCGCT GGAGATGCAA AAGGATCACA TCGCCGACTA TGCGCCGGTC   3000
   TGCGGTGCCC CCGGTTCTCC GGCCGGCGGT GGCACCTCTT CCGGTGGATC CGGTGGCGCG   3060
   GGCAGCGGTG CCAGCGGCGG CGATGACATT CATGGAGGAC ACGGCAGCGA ACGCAATCAG   3120
   CAGCGGTACG TGGGCGAGTA CTCCAACATA CCGACCGACT ATGCAGAGGT GTCCAGTTTT   3180
   GGCAAGGCAC CCAGCGAGTA TGGTCGGCAT GGCAACGCCT CCCCGGCCCC TTATGCCACC   3240
   TCTTCGATCC TGAGTCCCCA CCAGCAGCAA CAGCAGCAGC AGCCGCGTTA TCAACAGCGA   3300
   CCAGTGCCCG GCTATGGGCT CCAGCGCCCA ATGCACCCAC ACTACCAGCA GCAGCAGCAT   3360
   CAGCAGCAAC AGGCGCAGCA GACGCACCAG CAACACCAGG CTCTCCAGCA GCACCAGCAA   3420
   CTGCCACCCA GCAACATCTA CCAGCAGATG TCCACCACCA GCGAGATATA CCCCACGAAC   3480
   ACGGGTCCTT CGCGCTCTGT CTACTCTGAG CAGTATTACT ACCCCAAGGA CAAGCAGAGA   3540
   CACATCCACA TCACCGAGAA CAAGCTGAGC AACTGCCACA CCTATGAGGC GGCTCCTGGC   3600
   GCCAAGCAGT CCTCGCCGAT ATCCTCGCAG TTCGCCAGCG TGAGGCGGCA GCAGCTGCCG   3660
   CCCAACTGCA GCATCGGCAG GGAAAGTGCC CGCTTCAAGG TGCTAAACAC GGATCAGGGC   3720
   AAGAACCAGC AGAATCTCCT GGATCTCGAC GGCTCCTCGA TGTGCTACAA CGGTCTGGCA   3780
   GACTCGGGCT GCGGTGGATC TCCCTCCCCG ATGGCCATGC TGATGTCGCA CGAGGACGAG   3840
   CACGCGCTGT ACCACACGGC GGATGGGGAT CTGGACGACA TGGAACGACT GTACGTCAAG   3900
   GTGGACGAGC AGCAGCCTCC ACAGCAGCAG CAGCAGCTGA TTCCCCTGGT CCCACAGCAT   3960
   CCGGCGGAAG GTCACCTGCA GTCCTGGCGG AATCAGAGCA CGCGGAGCAG TCGGAAGAAC   4020
   GGCCAGGAAT GCATCAAGGA ACCCAGCGAG TTGATCTACG CTCCGGGAAG CGTGGCCAGC   4080
   GAACGGAGCC TCCTCAGCAA CTCGGGTAGC GGCACCAGCA GCCAGCCAGC TGGCCACAAT   4140
   GTCTGA                                                              4146
(2) INFORMATION FOR SEQ ID NO:4:
   (i) SEQUENCE CHARACTERISTICS:
      (A) LENGTH: 1381 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 3894 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:5:
   ATGTACTATC TAGGTTTTTA CCACACTCAC ACACACACAC ACACATACAT AAATTTTGAT      60
   AAAATTCCTA ATGCCTCAAA TCTCGCTCCC GTGATAATCG AACATCCCAT CGATGTGGTG     120
   GTATCTAGGG GATCGCCAGC AACCCTCAAC TGTGGTGCAA AGCCATCTAC CGCCAAAATC     180
   ACATGGTACA AGGATGGACA GCCCGTAATC ACGAATAAGG AGCAAGTGAA CAGCCACCGG 240
   ATTGTTCTCG ACACGGGATC CCTGTTTCTT CTGAAAGTGA ATAGTGGAAA AAACGGAAAA 300
   GACAGCGATG CGGGAGCGTA CTATTGTGTG GCCAGCAACG AGCACGGAGA AGTGAAGTCG 360
   AACGAAGGAT CGTTAAAATT GGCGATGCTT CGCGAAGACT TTCGAGTTCG GCCAAGAACA 420
   GTTCAGGCTC TTGGTGGAGA GATGGCCGTT CTGGAATGCA GTCCGCCACG TGGATTCCCG 480
   GAGCCGGTTG TGAGCTGGCG GAAAGACGAC AAAGAGCTCC GAATTCAAGA CATGCCACGA 540
   TACACTCTAC ACTCTGACGG AAACCTCATC ATTGATCCGG TCGATCGAAG CGATTCTGGT 600
   ACTTATCAGT GTGTTGCCAA CAACATGGTC GGAGAACGGG TGTCCAATCC CGCAAGATTG 660
   AGTGTCTTTG AGAAACCAAA GTTTGAGCAA GAACCCAAGG ACATGACGGT CGACGTCGGA 720
   GCCGCAGTGC TGTTTGATTG TCGTGTGACT GGAGATCCTC AACCACAAAT TACGTGGAAA 780
   CGCAAAAATG AGCCGATGCC AGTTACACGT GCATACATTG CCAAGGATAA TCGGGGGTTG 840
   AGAATCGAAA GAGTTCAACC ATCAGACGAA GGTGAATACG TTTGCTATGC ACGAAATCCA 900
   GCGGGAACTC TTGAAGCATC TGCACATCTT CGTGTCCAGG CACCTCCATC CTTCCAGACA 960
   AAACCAGCAG ACCAGTCAGT TCCAGCTGGA GGCACGGCAA CTTTTGAATG CACCTTGGTC 1020
   GGTCAACCGA GTCCCGCCTA TTTTTGGAGC AAGGAAGGCC AACAGGATCT TCTTTTCCCA 1080
   AGTTATGTGT CCGCTGATGG TAGAACGAAA GTTTCACCAA CTGGAACATT GACAATTGAG 1140
   GAAGTTCGTC AAGTTGATGA GGGAGCTTAT GTGTGCGCTG GAATGAACTC GGCAGGAAGC 1200
   TCGTTGAGCA AGGCAGCTTT GAAAGCAACA TTTGAAACCA AAGGCCGTGT CCAAAAAAAA 1260
   AAGAGCAAAA TGGGCAAACA GAAACAAAAA AATGTTCAAT CAATTATCAA ATATTTAATT 1320
   TCAGCCGTGA CCGGAAACAC ACCCGCCAAA CCACCACCAA CAATCGAGCA TGGTCATCAA 1380
   AATCAGACCC TTATGGTTGG ATCATCAGCC ATCCTTCCAT GTCAGGCTAG CGGAAAACCA 1440
   ACTCCAGGAA TATCATGGCT CAGGGATGGG CTACCTATTG ACATTACAGA TAGTCGTATC 1500
   AGTCAACATT CAACGGGAAG TCTACATATT GCCGATTTAA AGAAACCTGA CACCGGAGTT 1560
   TACACTTGCA TTGCGAAGAA CGAGGATGGA GAGTCAACAT GGTCGGCATC TCTGACTGTT 1620
   GAAGATCACA CTAGCAATGC ACAATTTGTT CGGATGCCGG ATCCATCGAA CTTCCCGTCT 1680
   TCTCCAACGC AACCCATTAT TGTCAATGTC ACTGATACCG AAGTAGAGCT CCACTGGAAT 1740
   GCTCCCTCCA CATCTGGCGC AGGACCAATC ACTGGTTATA TCATTCAGTA CTACAGTCCA 1800
   GACCTCGGAC AGACGTGGTT TAACATTCCA GACTACGTGG CATCTACTGA ATATAGAATA 1860
   AAGGGTCTGA AACCATCTCA CTCGTATATG TTTGTGATTC GAGCAGAAAA TGAGAAAGGT 1920
   ATTGGAACGC CGAGTGTGTC GTCGGCTCTC GTTACCACTA GCAAGCCAGC AGCTCAAGTT 1980
   GCGCTTTCTG ACAAGAACAA AATGGACATG GCCATCGCTG AGAAGAGACT CACTTCGGAA 2040
   CAACTCATAA AACTCGAGGA AGTGAAGACT ATTAATTCTA CGGCCGTTCG TTTGTTCTGG 2100
   AAGAAGAGGA AACTTGAAGA GCTGATTGAT GGTTACTACA TCAAGTGGAG AGGGCCTCCA 2160
   AGAACCAATG ATAATCAATA CGTGAATGTG ACCAGCCCTA GCACCGAAAA CTATGTTGTT 2220
   TCAAATTTAA TGCCATTCAC CAACTATGAG TTTTTCGTGA TTCCTTATCA TTCCGGAGTT 2280
   CATAGTATTC ATGGAGCACC GAGTAATTCC ATGGACGTGT TGACCGCCGA AGCTCCACCT 2340
   TCATTGCCAC CAGAGGATGT GCGAATCCGT ATGCTCAACC TGACCACTCT TCGTATCTCT 2400
   TGGAAAGCAC CAAAAGCCGA CGGCATCAAC GGAATTCTCA AAGGATTCCA AATTGTTATT 2460
   GTTGGTCAAG CGCCCAACAA CAATCGGAAC ATCACTACAA ACGAGAGAGC TGCCAGTGTT   2520
   ACTCTGTTCC ATTTAGTGAC TGGAATGACG TATAAAATTC GTGTAGCGGC TAGAAGCAAT   2580
   GGTGGAGTTG GAGTCTCACA TGGAACGAGT GAAGTCATCA TGAATCAAGA CACGCTGGAA   2640
   AAACACCTTG CTGCTCAACA AGAAAACGAA TCATTTTTGT ATGGGCTGAT CAATAAATCT   2700
   CATGTTCCTG TGATTGTCAT TGTTGCAATT CTGATTATTT TCGTAGTCAT CATTATAGCC   2760
   TATTGTTACT GGAGGAATAG CAGAAACAGT GATGGAAAGG ATCGAAGTTT TATAAAGATC   2820
   AATGATGGAA GTGTTCATAT GGCTTCGAAT AATCTTTGGG ATGTTGCACA AAATCCGAAT   2880
   CAGAATCCAA TGTACAACAC TGCTGGAAGA ATGACTATGA ACAATAGAAA TGGCCAGGCT   2940
   CTCTATTCGC TGACACCAAA TGCGCAAGAC TTTTTCAACA ATTGTGATGA CTACAGTGGA   3000
   ACGATGCACA GACCAGGATC CGAGCATCAC TATCATTATG CTCAACTGAC TGGCGGACCT   3060
   GGTAATGCGA TGTCTACTTT TTATGGAAAC CAATATCACG ATGATCCATC TCCATATGCC   3120
   ACCACAACAC TGGTCCTGTC GAACCAACAA CCAGCTTGGC TCAATGACAA AATGCTTCGC   3180
   GCGCCAGCAA TGCCAACAAA TCCCGTGCCA CCAGAGCCAC CGGCGCGATA TGCAGATCAT   3240
   ACCGCTGGAA GACGATCTCG ATCGAGCCGT GCATCCGATG GGAGAGGAAC TCTGAATGGC   3300
   GGACTCCATC ACCGGACTAG CGGAAGTCAA CGGTCGGATA GTCCACCTCA CACAGATGTG   3360
   AGCTATGTTC AGCTTCACTC ATCCGATGGA ACTGGTAGTA GTAAGGAAAG AACTGGGGAG   3420
   CGGAGAACAC CACCGAATAA GACTCTGATG GACTTTATTC CGCCACCACC TTCCAATCCA   3480
   CCACCACCTG GAGGGCACGT TTATGACACA GCAACTAGGC GTCAGTTGAA TCGTGGAAGT   3540
   ACTCCACGAG AAGACACCTA CGATTCGGTC AGTGACGGAG CTTTTGCTCG GGTTGATGTG   3600
   AATGCAAGGC CAACGAGTCG GAATCGGAAT TTGGGAGGAA GGCCGCTGAA AGGGAAACGA   3660
   GACGACGATA GTCAGCGGTC TTCGTTGATG ATGGACGATG ATGGTGGATC TTCTGAAGCT   3720
   GACGGGGAGA ACTCTGAAGG AGACGTTCCG CGTGGAGGTG TTAGAAAAGC AGTTCCTCGA   3780
   ATGGGTATCT CTGCAAGTAC GCTGGCTCAT AGTTGTTACG GGACAAACGG CACTGCTCAA   3840
   CGATTCCGGT CAATTCCACG TAACAATGGA ATCGTCACAC AAGAACAAAC TTGA         3894
(2) INFORMATION FOR SEQ ID NO:6:
   (i) SEQUENCE CHARACTERISTICS:
      (A) LENGTH: 1297 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:6:
   Met Tyr Tyr Leu Gly Phe Tyr His Thr His Thr His Thr His Thr Tyr
   1               5                   10                  15
   Ile Asn Phe Asp Lys Ile Pro Asn Ala Ser Asn Leu Ala Pro Val Ile
               20                  25                  30
   Ile Glu His Pro Ile Asp Val Val Val Ser Arg Gly Ser Pro Ala Thr
   35 40 45
   Leu Asn Cys Gly Ala Lys Pro Ser Thr Ala Lys Ile Thr Trp Tyr Lys
   50 55 60
   Asp Gly Gln Pro Val Ile Thr Asn Lys Glu Gln Val Asn Ser His Arg
   65 70 75 80
   Ile Val Leu Asp Thr Gly Ser Leu Phe Leu Leu Lys Val Asn Ser Gly
   85 90 95
   Lys Asn Gly Lys Asp Ser Asp Ala Gly Ala Tyr Tyr Cys Val Ala Ser
   100 105 110
   Asn Glu His Gly Glu Val Lys Ser Asn Glu Gly Ser Leu Lys Leu Ala
   115 120 125
   Met Leu Arg Glu Asp Phe Arg Val Arg Pro Arg Thr Val Gln Ala Leu
   130 135 140
   Gly Gly Glu Met Ala Val Leu Glu Cys Ser Pro Pro Arg Gly Phe Pro
   145 150 155 160
   Glu Pro Val Val Ser Trp Arg Lys Asp Asp Lys Glu Leu Arg Ile Gln
   165 170 175
   Asp Met Pro Arg Tyr Thr Leu His Ser Asp Gly Asn Leu Ile Ile Asp
   180 185 190
   Pro Val Asp Arg Ser Asp Ser Gly Thr Tyr Gln Cys Val Ala Asn Asn
   195 200 205
   Met Val Gly Glu Arg Val Ser Asn Pro Ala Arg Leu Ser Val Phe Glu
   210 215 220
   Lys Pro Lys Phe Glu Gln Glu Pro Lys Asp Met Thr Val Asp Val Gly
   225 230 235 240
   Ala Ala Val Leu Phe Asp Cys Arg Val Thr Gly Asp Pro Gln Pro Gln
   245 250 255
   Ile Thr Trp Lys Arg Lys Asn Glu Pro Met Pro Val Thr Arg Ala Tyr
   260 265 270
   Ile Ala Lys Asp Asn Arg Gly Leu Arg Ile Glu Arg Val Gln Pro Ser
   275 280 285
   Asp Glu Gly Glu Tyr Val Cys Tyr Ala Arg Asn Pro Ala Gly Thr Leu
   290 295 300
   Glu Ala Ser Ala His Leu Arg Val Gln Ala Pro Pro Ser Phe Gln Thr
   305 310 315 320
   Lys Pro Ala Asp Gln Ser Val Pro Ala Gly Gly Thr Ala Thr Phe Glu
   325 330 335
   Cys Thr Leu Val Gly Gln Pro Ser Pro Ala Tyr Phe Trp Ser Lys Glu
   340 345 350
   Gly Gln Gln Asp Leu Leu Phe Pro Ser Tyr Val Ser Ala Asp Gly Arg
   355 360 365
   Thr Lys Val Ser Pro Thr Gly Thr Leu Thr Ile Glu Glu Val Arg Gln
   370 375 380
   Val Asp Glu Gly Ala Tyr Val Cys Ala Gly Met Asn Ser Ala Gly Ser
   385 390 395 400
   Ser Leu Ser Lys Ala Ala Leu Lys Ala Thr Phe Glu Thr Lys Gly Arg
   405 410 415
   Val Gln Lys Lys Lys Ser Lys Met Gly Lys Gln Lys Gln Lys Asn Val
   420 425 430
   Gln Ser Ile Ile Lys Tyr Leu Ile Ser Ala Val Thr Gly Asn Thr Pro
   435 440 445
   Ala Lys Pro Pro Pro Thr Ile Glu His Gly His Gln Asn Gln Thr Leu
   450 455 460
   Met Val Gly Ser Ser Ala Ile Leu Pro Cys Gln Ala Ser Gly Lys Pro
   465 470 475 480
   Thr Pro Gly Ile Ser Trp Leu Arg Asp Gly Leu Pro Ile Asp Ile Thr
   485 490 495
   Asp Ser Arg Ile Ser Gln His Ser Thr Gly Ser Leu His Ile Ala Asp
   500 505 510
   Leu Lys Lys Pro Asp Thr Gly Val Tyr Thr Cys Ile Ala Lys Asn Glu
   515 520 525
   Asp Gly Glu Ser Thr Trp Ser Ala Ser Leu Thr Val Glu Asp His Thr
   530 535 540
   Ser Asn Ala Gln Phe Val Arg Met Pro Asp Pro Ser Asn Phe Pro Ser
   545 550 555 560
   Ser Pro Thr Gln Pro Ile Ile Val Asn Val Thr Asp Thr Glu Val Glu
   565 570 575
   Leu His Trp Asn Ala Pro Ser Thr Ser Gly Ala Gly Pro Ile Thr Gly
   580 585 590
   Tyr Ile Ile Gln Tyr Tyr Ser Pro Asp Leu Gly Gln Thr Trp Phe Asn
   595 600 605
   Ile Pro Asp Tyr Val Ala Ser Thr Glu Tyr Arg Ile Lys Gly Leu Lys
   610 615 620
   Pro Ser His Ser Tyr Met Phe Val Ile Arg Ala Glu Asn Glu Lys Gly
   625 630 635 640
   Ile Gly Thr Pro Ser Val Ser Ser Ala Leu Val Thr Thr Ser Lys Pro
   645 650 655
   Ala Ala Gln Val Ala Leu Ser Asp Lys Asn Lys Met Asp Met Ala Ile
   660 665 670
   Ala Glu Lys Arg Leu Thr Ser Glu Gln Leu Ile Lys Leu Glu Glu Val
   675 680 685
   Lys Thr Ile Asn Ser Thr Ala Val Arg Leu Phe Trp Lys Lys Arg Lys
   690 695 700
   Leu Glu Glu Leu Ile Asp Gly Tyr Tyr Ile Lys Trp Arg Gly Pro Pro
   705 710 715 720
   Arg Thr Asn Asp Asn Gln Tyr Val Asn Val Thr Ser Pro Ser Thr Glu
   725 730 735
   Asn Tyr Val Val Ser Asn Leu Met Pro Phe Thr Asn Tyr Glu Phe Phe
   740 745 750
   Val Ile Pro Tyr His Ser Gly Val His Ser Ile His Gly Ala Pro Ser
   755 760 765
   Asn Ser Met Asp Val Leu Thr Ala Glu Ala Pro Pro Ser Leu Pro Pro
   770 775 780
   Glu Asp Val Arg Ile Arg Met Leu Asn Leu Thr Thr Leu Arg Ile Ser
   785 790 795 800
   Trp Lys Ala Pro Lys Ala Asp Gly Ile Asn Gly Ile Leu Lys Gly Phe
   805 810 815
   Gln Ile Val Ile Val Gly Gln Ala Pro Asn Asn Asn Arg Asn Ile Thr
   820 825 830
   Thr Asn Glu Arg Ala Ala Ser Val Thr Leu Phe His Leu Val Thr Gly
   835 840 845
   Met Thr Tyr Lys Ile Arg Val Ala Ala Arg Ser Asn Gly Gly Val Gly
   850 855 860
   Val Ser His Gly Thr Ser Glu Val Ile Met Asn Gln Asp Thr Leu Glu
   865 870 875 880
   Lys His Leu Ala Ala Gln Gln Glu Asn Glu Ser Phe Leu Tyr Gly Leu
   885 890 895
   Ile Asn Lys Ser His Val Pro Val Ile Val Ile Val Ala Ile Leu Ile
   900 905 910
   Ile Phe Val Val Ile Ile Ile Ala Tyr Cys Tyr Trp Arg Asn Ser Arg
   915 920 925
   Asn Ser Asp Gly Lys Asp Arg Ser Phe Ile Lys Ile Asn Asp Gly Ser
   930 935 940
   Val His Met Ala Ser Asn Asn Leu Trp Asp Val Ala Gln Asn Pro Asn
   945 950 955 960
   Gln Asn Pro Met Tyr Asn Thr Ala Gly Arg Met Thr Met Asn Asn Arg
   965 970 975
   Asn Gly Gln Ala Leu Tyr Ser Leu Thr Pro Asn Ala Gln Asp Phe Phe
   980 985 990
   Asn Asn Cys Asp Asp Tyr Ser Gly Thr Met His Arg Pro Gly Ser Glu
   995 1000 1005
   His His Tyr His Tyr Ala Gln Leu Thr Gly Gly Pro Gly Asn Ala Met
   1010 1015 1020
   Ser Thr Phe Tyr Gly Asn Gln Tyr His Asp Asp Pro Ser Pro Tyr Ala
   1025 1030 1035 1040
   Thr Thr Thr Leu Val Leu Ser Asn Gln Gln Pro Ala Trp Leu Asn Asp
   1045 1050 1055
   Lys Met Leu Arg Ala Pro Ala Met Pro Thr Asn Pro Val Pro Pro Glu
   1060 1065 1070
   Pro Pro Ala Arg Tyr Ala Asp His Thr Ala Gly Arg Arg Ser Arg Ser
   1075 1080 1085
   Ser Arg Ala Ser Asp Gly Arg Gly Thr Leu Asn Gly Gly Leu His His
   1090 1095 1100
   Arg Thr Ser Gly Ser Gln Arg Ser Asp Ser Pro Pro His Thr Asp Val
   1105 1110 1115 1120
   Ser Tyr Val Gln Leu His Ser Ser Asp Gly Thr Gly Ser Ser Lys Glu
   1125 1130 1135
   Arg Thr Gly Glu Arg Arg Thr Pro Pro Asn Lys Thr Leu Met Asp Phe
   1140 1145 1150
   Ile Pro Pro Pro Pro Ser Asn Pro Pro Pro Pro Gly Gly His Val Tyr
   1155 1160 1165
   Asp Thr Ala Thr Arg Arg Gln Leu Asn Arg Gly Ser Thr Pro Arg Glu
   1170 1175 1180
   Asp Thr Tyr Asp Ser Val Ser Asp Gly Ala Phe Ala Arg Val Asp Val
   1185 1190 1195 1200
   Asn Ala Arg Pro Thr Ser Arg Asn Arg Asn Leu Gly Gly Arg Pro Leu
   1205 1210 1215
   Lys Gly Lys Arg Asp Asp Asp Ser Gln Arg Ser Ser Leu Met Met Asp
   1220 1225 1230
   Asp Asp Gly Gly Ser Ser Glu Ala Asp Gly Glu Asn Ser Glu Gly Asp
   1235 1240 1245
   Val Pro Arg Gly Gly Val Arg Lys Ala Val Pro Arg Met Gly Ile Ser
       1250                1255                1260
   Ala Ser Thr Leu Ala His Ser Cys Tyr Gly Thr Asn Gly Thr Ala Gln
   1265                1270                1275                1280
   Arg Phe Arg Ser Ile Pro Arg Asn Asn Gly Ile Val Thr Gln Glu Gln
                   1285                1290                1295
   Thr
(2) INFORMATION FOR SEQ ID NO:7:
   (i) SEQUENCE CHARACTERISTICS:
      (A) LENGTH: 4956 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:7:
   ATGAAATGGA AACATGTTCC TTTTTTGGTC ATGATATCAC TCCTCAGCTT ATCCCCAAAT     60
   CACCTGTTTC TGGCCCAGCT TATTCCAGAC CCTGAAGATG TAGAGAGGGG GAACGACCAC    120
   GGGACGCCAA TCCCCACCTC TGATAACGAT GACAATTCGC TGGGCTATAC AGGCTCCCGT    180
   CTTCGTCAGG AAGATTTTCC ACCTCGCATT GTTGAACACC CTTCAGACCT GATTGTCTCA    240
   AAAGGAGAAC CTGCAACTTT GAACTGCAAA GCTGAAGGCC GCCCCACACC CACTATTGAA    300
   TGGTACAAAG GGGGAGAGAG AGTGGAGACA GACAAAGATG ACCCTCGCTC ACACCGAATG    360
   TTGCTGCCGA GTGGATCTTT ATTTTTCTTA CGTATAGTAC ATGGACGGAA AAGTAGACCT    420
   GATGAAGGAG TCTATGTCTG TGTAGCAAGG AATTACCTTG GAGAGGCTGT GAGCCACAAT    480
   GCATCGCTGG AAGTAGCCAT ACTTCGGGAT GACTTCAGAC AAAACCCTTC GGATGTCATG    540
   GTTGCAGTAG GAGAGCCTGC AGTAATGGAA TGCCAACCTC CACGAGGCCA TCCTGAGCCC    600
   ACCATTTCAT GGAAGAAAGA TGGCTCTCCA CTGGATGATA AAGATGAAAG AATAACTATA    660
   CGAGGAGGAA AGCTCATGAT CACTTACACC CGTAAAAGTG ACGCTGGCAA ATATGTTTGT    720
   GTTGGTACCA ATATGGTTGG GGAACGTGAG AGTGAAGTAG CCGAGCTGAC TGTCTTAGAG    780
   AGACCATCAT TTGTGAAGAG ACCCAGTAAC TTGGCAGTAA CTGTGGATGA CAGTGCAGAA    840
   TTTAAATGTG AGGCCCGAGG TGACCCTGTA CCTACAGTAC GATGGAGGAA AGATGATGGA    900
   GAGCTGCCCA AATCCAGATA TGAAATCCGA GATGATCATA CCTTGAAAAT TAGGAAGGTG    960
   ACAGCTGGTG ACATGGGTTC ATACACTTGT GTTGCAGAAA ATATGGTGGG CAAAGCTGAA   1020
   GCATCTGCTA CTCTGACTGT TCAAGAACCT CCACATTTTG TTGTGAAACC CCGTGACCAG   1080
   GTTGTTGCTT TGGGACGGAC TGTAACTTTT CAGTGTGAAG CAACCGGAAA TCCTCAACCA   1140
   GCTATTTTCT GGAGGAGAGA AGGGAGTCAG AATCTACTTT TCTCATATCA ACCACCACAG   1200
   TCATCCAGCC GATTTTCAGT CTCCCAGACT GGCGACCTCA CAATTACTAA TGTCCAGCGA   1260
   TCTGATGTTG GTTATTACAT CTGCCAGACT TTAAATGTTG CTGGAAGCAT CATCACAAAG   1320
   GCATATTTGG AAGTTACAGA TGTGATTGCA GATCGGCCTC CCCCAGTTAT TCGACAAGGT   1380
   CCTGTGAATC AGACTGTAGC CGTGGATGGC ACTTTCGTCC TCAGCTGTGT GGCCACAGGC 1440
   AGTCCAGTGC CCACCATTCT GTGGAGAAAG GATGGAGTCC TCGTTTCAAC CCAAGACTCT 1500
   CGAATCAAAC AGTTGGAGAA TGGAGTACTG CAGATCCGAT ATGCTAAGCT GGGTGATACT 1560
   GGTCGGTACA CCTGCATTGC ATCAACCCCC AGTGGTGAAG CAACATGGAG TGCTTACATT 1620
   GAAGTTCAAG AATTTGGAGT TCCAGTTCAG CCTCCAAGAC CTACTGACCC AAATTTAATC 1680
   CCTAGTGCCC CATCAAAACC TGAAGTGACA GATGTCAGCA GAAATACAGT CACATTATCG 1740
   TGGCAACCAA ATTTGAATTC AGGAGCAACT CCAACATCTT ATATTATAGA AGCCTTCAGC 1800
   CATGCATCTG GTAGCAGCTG GCAGACCGTA GCAGAGAATG TGAAAACAGA AACATCTGCC 1860
   ATTAAAGGAC TCAAACCTAA TGCAATTTAC CTTTTCCTTG TGAGGGCAGC TAATGCATAT 1920
   GGAATTAGTG ATCCAAGCCA AATATCAGAT CCAGTGAAAA CACAAGATGT CCTACCAACA 1980
   AGTCAGGGGG TGGACCACAA GCAGGTCCAG AGAGAGCTGG GAAATGCTGT TCTGCACCTC 2040
   CACAACCCCA CCGTCCTTTC TTCCTCTTCC ATCGAAGTGC ACTGGACAGT AGATCAACAG 2100
   TCTCAGTATA TACAAGGATA TAAAATTCTC TATCGGCCAT CTGGAGCCAA CCACGGAGAA 2160
   TCAGACTGGT TAGTTTTTGA AGTGAGGACG CCAGCCAAAA ACAGTGTGGT AATCCCTGAT 2220
   CTCAGAAAGG GAGTCAACTA TGAAATTAAG GCTCGCCCTT TTTTTAATGA ATTTCAAGGA 2280
   GCAGATAGTG AAATCAAGTT TGCCAAAACC CTGGAAGAAG CACCCAGTGC CCCACCCCAA 2340
   GGTGTAACTG TATCCAAGAA TGATGGAAAC GGAACTGCAA TTCTAGTTAG TTGGCAGCCA 2400
   CCTCCAGAAG ACACTCAAAA TGGAATGGTC CAAGAGTATA AGGTTTGGTG TCTGGGCAAT 2460
   GAAACTCGAT ACCACATCAA CAAAACAGTG GATGGTTCCA CCTTTTCCGT GGTCATTCCC 2520
   TTTCTTGTTC CTGGAATCCG ATACAGTGTG GAAGTGGCAG CCAGCACTGG GGCTGGGTCT 2580
   GGGGTAAAGA GTGAGCCTCA GTTCATCCAG CTGGATGCCC ATGGAAACCC TGTGTCACCT 2640
   GAGGACCAAG TCAGCCTCGC TCAGCAGATT TCAGATGTGG TGAAGCAGCC GGCCTTCATA 2700
   GCAGGTATTG GAGCAGCCTG TTGGATCATC CTCATGGTCT TCAGCATCTG GCTTTATCGA 2760
   CACCGCAAGA AGAGAAACGG ACTTACTAGT ACCTACGCGG GTATCAGAAA AGTCCCGTCT 2820
   TTTACCTTCA CACCAACAGT AACTTACCAG AGAGGAGGCG AAGCTGTCAG CAGTGGAGGG 2880
   AGGCCTGGAC TTCTCAACAT CAGTGAACCT GCCGCGCAGC CATGGCTGGC AGACACGTGG 2940
   CCTAATACTG GCAACAACCA CAATGACTGC TCCATCAGCT GCTGCACGGC AGGCAATGGA 3000
   AACAGCGACA GCAACCTCAC TACCTACAGT CGCCCAGCTG ATTGTATAGC AAATTATAAC 3060
   AACCAACTGG ATAACAAACA AACAAATCTG ATGCTCCCTG AGTCAACTGT TTATGGTGAT 3120
   GTGGACCTTA GTAACAAAAT CAATGAGATG AAAACCTTCA ATAGCCCAAA TCTGAAGGAT 3180
   GGGCGTTTTG TCAATCCATC AGGGCAGCCT ACTCCTTACG CCACCACTCA GCTCATCCAG 3240
   TCAAACCTCA GCAACAACAT GAACAATGGC AGCGGGGACT CTGGCGAGAA GCACTGGAAA 3300
   CCACTGGGAC AGCAGAAACA AGAAGTGGCA CCAGTTCAGT ACAACATCGT GGAGCAAAAC 3360
   AAGCTGAACA AAGATTATCG AGCAAATGAC ACAGTTCCTC CAACTATCCC ATACAACCAA 3420
   TCATACGACC AGAACACAGG AGGATCCTAC AACAGCTCAG ACCGGGGCAG TAGTACATCT 3480
   GGGAGTCAGG GGCACAAGAA AGGGGCAAGA ACACCCAAGG TACCAAAACA GGGTGGCATG 3540
   AACTGGGCAG ACCTGCTTCC TCCTCCCCCA GCACATCCTC CTCCACACAG CAATAGCGAA 3600
   GAGTACAACA TTTCTGTAGA TGAAAGCTAT GACCAAGAAA TGCCATGTCC CGTGCCACCA 3660
   GCAAGGATGT ATTTGCAACA AGATGAATTA GAAGAGGAGG AAGATGAACG AGGCCCCACT 3720
   CCCCCTGTTC GGGGAGCAGC TTCTTCTCCA GCTGCCGTGT CCTATAGCCA TCAGTCCACT 3780
   GCCACTCTGA CTCCCTCCCC ACAGGAAGAA CTCCAGCCCA TGTTACAGGA TTGTCCAGAG 3840
   GAGACTGGCC ACATGCAGCA CCAGCCCGAC AGGAGACGGC AGCCTGTGAG TCCTCCTCCA 3900
   CCACCACGGC CGATCTCCCC TCCACATACC TATGGCTACA TTTCAGGACC CCTGGTCTCA 3960
   GATATGGATA CGGATGCGCC AGAAGAGGAA GAAGACGAAG CCGACATGGA GGTAGCCAAG 4020
   ATGCAAACCA GAAGGCTTTT GTTACGTGGG CTTGAGCAGA CACCTGCCTC CAGTGTTGGG 4080
   GACCTGGAGA GCTCTGTCAC GGGGTCCATG ATCAACGGCT GGGGCTCAGC CTCAGAGGAG 4140
   GACAACATTT CCAGCGGACG CTCCAGTGTT AGTTCTTCGG ACGGCTCCTT TTTCACTGAT 4200
   GCTGACTTTG CCCAGGCAGT CGCAGCAGCG GCAGAGTATG CTGGTCTGAA AGTAGCACGA 4260
   CGGCAAATGC AGGATGCTGC TGGCCGTCGA CATTTTCATG CGTCTCAGTG CCCTAGGCCC 4320
   ACAAGTCCCG TGTCTACAGA CAGCAACATG AGTGCCGCCG TAATGCAGAA AACCAGACCA 4380
   GCCAAGAAAC TGAAACACCA GCCAGGACAT CTGCGCAGAG AAACCTACAC AGATGATCTT 4440
   CCACCACCTC CTGTGCCGCC ACCTGCTATA AAGTCACCTA CTGCCCAATC CAAGACACAG 4500
   CTGGAAGTAC GACCTGTAGT GGTGCCAAAA CTCCCTTCTA TGGATGCAAG AACAGACAGA 4560
   TCATCAGACA GAAAAGGAAG CAGTTACAAG GGGAGAGAAG TGTTGGATGG AAGACAGGTT 4620
   GTTGACATGC GAACAAATCC AGGTGATCCC AGAGAAGCAC AGGAACAGCA AAATGACGGG 4680
   AAAGGACGTG GAAACAAGGC AGCAAAACGA GACCTTCCAC CAGCAAAGAC TCATCTCATC 4740
   CAAGAGGATA TTCTACCTTA TTGTAGACCT ACTTTTCCAA CATCAAATAA TCCCAGAGAT 4800
   CCCAGTTCCT CAAGCTCAAT GTCATCAAGA GGATCAGGAA GCAGACAAAG AGAACAAGCA 4860
   AATGTAGGTC GAAGAAATAT TGCAGAAATG CAGGTACTTG GAGGATATGA AAGAGGAGAA 4920
   GATAATAATG AAGAATTAGA GGAAACTGAA AGCTGA 4956
(2) INFORMATION FOR SEQ ID NO:8:
   (i) SEQUENCE CHARACTERISTICS:
      (A) LENGTH: 1651 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 1300 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: misc_feature
      (B) LOCATION: 855..1187
      (D) OTHER INFORMATION: /note= "N signifies gap in sequence"
   (xi) SEQUENCE DESCRIPTION: SEQ ID NO:9:
   CAGATTGTTG CTCAAGGTCG AACAGTGACA TTTCCCTGTG AAACTAAAGG AAACCCACAG    60
   CCAGCTGTTT TTTGGCAGAA AGAAGGCAGC CAGAACCTAC TTTTCCCAAA CCAACCCCAG   120
   CAGCCCAACA GTAGATGCTC AGTGTCACCA ACTGGAGACC TCACAATCAC CAACATTCAA   180
   CGTTCCGACG CGGGTTACTA CATCTGCCAG GCTTTAACTG TGGCAGGAAG CATTTTAGCA   240
   AAAGCTCAAC TGGAGGTTAC TGATGTTTTG ACAGATAGAC CTCCACCTAT AATTCTACAA   300
   GGCCCAGCCA ACCAAACGCT GGCAGTGGAT GGTACAGCGT TACTGAAATG TAAAGCCACT   360
   GGTGATCCTC TTCCTGTAAT TAGCTGGTTA AAGGAGGGAT TTACTTTTCC GGGTAGAGAT   420
   CCAAGAGCAA CAATTCAAGA GCAAGGCACA CTGCAGATTA AGAATTTACG GATTTCTGAT   480
   ACTGGCACTT ATACTTGTGT GGCTACAAGT TCAAGTGGAG AGGCTTCCTG GAGTGCAGTG   540
   CTGGATGTGA CAGAGTCTGG AGCAACAATC AGTAAAAACT ATGATTTAAG TGACCTGCCA   600
   GGGCCACCAT CCAAACCGCA AGTCACTGAT GTTACTAAGA ACAGTGTCAC CTTGTCCTGG   660
   CAGCCAGGTA CCCCTGGAAC CCTTCCAGCA AGTGCATATA TCATTGAGGC TTTCAGCCAA   720
   TCAGTGAGCA ACAGCTGGCA GACCGTGGCA AACCATGTAA AGACCACCCT CTATACTGTA   780
   AGAGGACTGC GGCCCAATAC AATCTACTTA TTCATGGTCA GAGCGATCAA CCCCAAGGTY   840
   TCAGTGACCC AAGTNAAACC ACAGAAAAAC AATGGATCCA CTTGGGCCAA TGTCCCTCTA     900
   CCTCCCCCCC CAGTCCAGCC CCTTCCTGGC ACGGAGCTGG AACACTATGC AGTGGAACAA     960
   CAAGAAAATG GCTATGACAG TGATAGCTGG TGCCCACCAT TGCCAGTACA AACTTACTTA    1020
   CACCAAGGTC TGGAAGATGA ACTGGAAGAA GATGATGATA GGGTCCCAAC ACCTCCTGTT    1080
   CGAGGCGTGG CTTCTTCTCC TGCTATCTCC TTTGGACAGC AGTCCACTGC AACTCTTACT    1140
   CCATCCCCAC GGGAAGAGAT GCAACCCATG CTGCAGGCTT CACCTNTTTA CCTCCTCTCA    1200
   AAGACCTCGA CCTACCAGCC CATTTTCTAC TGACAGTAAC ACCAGTGCAG CCCTGAGTCA    1260
   AAGTCAGAGG CCTCGGCCCA CTAAAAAACA CAAGGGAGGG                          1300
(2) INFORMATION FOR SEQ ID NO:10:
   (i) SEQUENCE CHARACTERISTICS:
      (A) LENGTH: 434 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 285..396
      (D) OTHER INFORMATION: /note= "Xaa signifies gap in sequence"
   (xi) SEQUENCE DESCRIPTION: SEQ ID NO:10:
   Gln Ile Val Ala Gln Gly Arg Thr Val Thr Phe Pro Cys Glu Thr Lys
   1 5 10 15
   Gly Asn Pro Gln Pro Ala Val Phe Trp Gln Lys Glu Gly Ser Gln Asn
   20 25 30
   Leu Leu Phe Pro Asn Gln Pro Gln Gln Pro Asn Ser Arg Cys Ser Val
   35 40 45
   Ser Pro Thr Gly Asp Leu Thr Ile Thr Asn Ile Gln Arg Ser Asp Ala
   50 55 60
   Gly Tyr Tyr Ile Cys Gln Ala Leu Thr Val Ala Gly Ser Ile Leu Ala
   65 70 75 80
   Lys Ala Gln Leu Glu Val Thr Asp Val Leu Thr Asp Arg Pro Pro Pro
   85 90 95
   Ile Ile Leu Gln Gly Pro Ala Asn Gln Thr Leu Ala Val Asp Gly Thr
   100 105 110
   Ala Leu Leu Lys Cys Lys Ala Thr Gly Asp Pro Leu Pro Val Ile Ser
   115 120 125
   Trp Leu Lys Glu Gly Phe Thr Phe Pro Gly Arg Asp Pro Arg Ala Thr
   130 135 140
   Ile Gln Glu Gln Gly Thr Leu Gln Ile Lys Asn Leu Arg Ile Ser Asp
   145 150 155 160
   Thr Gly Thr Tyr Thr Cys Val Ala Thr Ser Ser Ser Gly Glu Ala Ser
   165 170 175
   Trp Ser Ala Val Leu Asp Val Thr Glu Ser Gly Ala Thr Ile Ser Lys
   180 185 190
   Asn Tyr Asp Leu Ser Asp Leu Pro Gly Pro Pro Ser Lys Pro Gln Val
   195 200 205
   Thr Asp Val Thr Lys Asn Ser Val Thr Leu Ser Trp Gln Pro Gly Thr
   210 215 220
   Pro Gly Thr Leu Pro Ala Ser Ala Tyr Ile Ile Glu Ala Phe Ser Gln
   225 230 235 240
   Ser Val Ser Asn Ser Trp Gln Thr Val Ala Asn His Val Lys Thr Thr
   245 250 255
   Leu Tyr Thr Val Arg Gly Leu Arg Pro Asn Thr Ile Tyr Leu Phe Met
   260 265 270
   Val Arg Ala Ile Asn Pro Lys Val Ser Val Thr Gln Xaa Lys Pro Gln
   275 280 285
   Lys Asn Asn Gly Ser Thr Trp Ala Asn Val Pro Leu Pro Pro Pro Pro
   290 295 300
   Val Gln Pro Leu Pro Gly Thr Glu Leu Glu His Tyr Ala Val Glu Gln
   305 310 315 320
   Gln Glu Asn Gly Tyr Asp Ser Asp Ser Trp Cys Pro Pro Leu Pro Val
   325 330 335
   Gln Thr Tyr Leu His Gln Gly Leu Glu Asp Glu Leu Glu Glu Asp Asp
   340 345 350
   Asp Arg Val Pro Thr Pro Pro Val Arg Gly Val Ala Ser Ser Pro Ala
   355 360 365
   Ile Ser Phe Gly Gln Gln Ser Thr Ala Thr Leu Thr Pro Ser Pro Arg
   370 375 380
   Glu Glu Met Gln Pro Met Leu Gln Ala Ser Pro Xaa Phe Thr Ser Ser
   385 390 395 400
   Gln Arg Pro Arg Pro Thr Ser Pro Phe Ser Thr Asp Ser Asn Thr Ser
   405 410 415
   Ala Ala Leu Ser Gln Ser Gln Arg Pro Arg Pro Thr Lys Lys His Lys
   420 425 430
   Gly Gly
(2) INFORMATION FOR SEQ ID NO:11:
   (i) SEQUENCE CHARACTERISTICS:
      (A) LENGTH: 444 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:11:
   GCCCAGGCAG TTGCTGCAGC TGCGGAGTAT GCGGGCCTGA AAGTGGCTCG CCGCCAAATG    60
   CAAGATGCTG CTGGCCGCCG CCACTTCCAT GCCTCTCAGT GCCCAAGGCC CACGAGTCCT   120
   GTGTCCACAG ACAGCAACAT GAGTGCTGTT GTGATCCAGA AAGCCAGACC CGCCAAGAAG   180
   CAGAAACACC AGCCAGGACA TCTGCGCAGG GAAGCCTACG CAGATGATCT TCCACCCCCT   240
   CCAGTGCCAC CACCTGCTAT AAAATCGCCC ACTGTCCAGT CCAAGGCACA GCTGGAGGTA   300
   CGGCCTGTCA TGGTGCCAAA ACTCGCGTCT ATAGAAGCAA GGACAGATAG ATCGTCAGAC   360
   AGAAAAGGAG GCAGTTACAA GGGGAGAGAA GCTCTGGATG GAAGACAAGT CACTGACCTG   420
   CGAACAAATC CAAGTGACCC CAGA                                          444
(2) INFORMATION FOR SEQ ID NO:12:
   (i) SEQUENCE CHARACTERISTICS:
      (A) LENGTH: 148 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: *SEQ* ID NO:12:
   Ala Gln Ala Val Ala Ala Ala Ala Glu Tyr Ala Gly Leu Lys Val Ala
   1               5                   10                  15
   Arg Arg Gln Met Gln Asp Ala Ala Gly Arg Arg His Phe His Ala Ser
               20                  25                  30
   Gln Cys Pro Arg Pro Thr Ser Pro Val Ser Thr Asp Ser Asn Met Ser
           35                  40                  45
   Ala Val Val Ile Gln Lys Ala Arg Pro Ala Lys Lys Gln Lys His Gln
       50                  55                  60
   Pro Gly His Leu Arg Arg Glu Ala Tyr Ala Asp Asp Leu Pro Pro Pro
   65                  70                  75                  80
   Pro Val Pro Pro Pro Ala Ile Lys Ser Pro Thr Val Gln Ser Lys Ala
                   85                  90                  95
   Gln Leu Glu Val Arg Pro Val Met Val Pro Lys Leu Ala Ser Ile Glu
               100                 105                 110
   Ala Arg Thr Asp Arg Ser Ser Asp Arg Lys Gly Gly Ser Tyr Lys Gly
           115                 120                 125
   Arg Glu Ala Leu Asp Gly Arg Gln Val Thr Asp Leu Arg Thr Asn Pro
       130                 135                 140
   Ser Asp Pro Arg
   145